(19) Europäisches Patentamt / European Patent Office / Office européen des brevets

(11) **EP 4 671 764 A1**

(12) **EUROPEAN PATENT APPLICATION**
published in accordance with Art. 153(4) EPC

(43) Date of publication:
**31.12.2025 Bulletin 2026/01**

(21) Application number: **23924201.9**

(22) Date of filing: **28.11.2023**

(51) International Patent Classification (IPC):
**G01N 33/552** (2006.01)   **B01J 20/24** (2006.01)
**C01B 33/12** (2006.01)   **C01B 37/00** (2006.01)
**C07K 16/00** (2006.01)   **C07K 17/14** (2006.01)
**C12M 1/34** (2006.01)

(52) Cooperative Patent Classification (CPC):
**B01J 20/24; C01B 33/12; C01B 37/00;
C07K 16/00; C07K 17/14; C12M 1/34; G01N 33/552**

(86) International application number:
**PCT/JP2023/042614**

(87) International publication number:
**WO 2024/176557 (29.08.2024 Gazette 2024/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.02.2023 JP 2023027780**

(71) Applicants:
• **Ohara, Inc.**
  **Sagamihara-shi, Kanagawa 252-5286 (JP)**
• **National University Corporation
  Nagaoka University of Technology
  Nagaoka-shi
  Niigata 940-2188 (JP)**

• **Kyushu Institute of Technology
  Kitakyushu-shi, Fukuoka 804-8550 (JP)**

(72) Inventors:
• **KIMURA Reo
  Nagaoka-shi, Niigata 940-2188 (JP)**
• **CHATANI Sunao
  Wakayama-shi, Wakayama 640-8404 (JP)**
• **INUI Masahiko
  Wakayama-shi, Wakayama 640-8404 (JP)**
• **MOTOZUKA Satoshi
  Kitakyusyu-shi, Fukuoka 804-8550 (JP)**
• **TAGAYA Motohiro
  Nagaoka-shi, Niigata 940-2188 (JP)**

(74) Representative: **Maiwald GmbH
  Elisenhof
  Elisenstraße 3
  80335 München (DE)**

(54) **PROTEIN IMMOBILIZATION CARRIER, PROTEIN IMMOBILIZATION CARRIER DISPERSION LIQUID, PROTEIN IMMOBILIZATION CARRIER HYDRATE, PROTEIN SUPPORT COMPLEX, AND ANTIBODY TESTING KIT**

(57)     Provided are: a protein immobilization carrier that can be produced with a simple method, exhibits little degeneration of protein that has been adsorbed, and demonstrates a high protein-binding activity; and a protein immobilization carrier dispersion liquid, a protein immobilization carrier hydrate, a protein support complex, and an antibody testing kit that use the protein immobilization carrier. The protein immobilization carrier is composed of an amorphous silica-based compound containing silicon (Si), oxygen (O), and chlorine (Cl). When, in a solid-state $^{29}$Si-NMR spectrum of the carrier, the peak area derived from $Si(OSi)_4$ is noted as $Q_4$, the peak area derived from $HO\text{-}Si(OSi)_3$ is noted as $Q_3$, and the peak area derived from $(HO)_2\text{-}Si(OSi)_2$ is noted as $Q_2$, $(Q_2 + Q_3) / (Q_2 + Q_3 + Q_4)$ is 0.30 or less. The molar concentration of chlorine atoms in the carrier as measured by X-ray fluorescence (XRF) analysis is 0.01 mol% or more.

FIG. 1A

EP 4 671 764 A1

# FIG. 1B

# FIG. 1C

**Description**

TECHNICAL FIELD

The present invention relates to a protein immobilization carrier, a protein immobilization carrier dispersion, a protein immobilization carrier hydrate, a protein-carrying complex, and an antibody test kit.

BACKGROUND ART

A protein immobilization carrier has been studied, which is for immobilizing a protein, such as an antibody protein that has the ability to specifically bind to a target substance, such as an antigen, which can therefore be immobilized on the carrier though the protein. Such a protein immobilization carrier is required to efficiently immobilize a protein that serves as a biding ligand for a target substance.

For example, Patent Document 1 discloses a protein immobilization carrier including a mesoporous zirconia particle having mesopores in which proteins can be adsorbed and immobilized.

Patent Document 2 discloses a silica-based biomolecule carrier for delivering a biomolecule into cells, which includes a silica-based porous core, such as a mesoporous silica nanoparticle; a first bioactive moiety; a second bioactive moiety that is different from but functionally associated with the first bioactive moiety; and linkers which separately conjugate each of the first bioactive moiety and the second bioactive moiety to the silica-based porous core.

Citation List

Patent Document

   Patent Document 1: Japanese Unexamined Patent Application, Publication No. 2017-047365
   Patent Document 2: Japanese Unexamined Patent Application, Publication No. 2018-533543

DISCLOSURE OF THE INVENTION

Problems to be Solved by the Invention

Unfortunately, the protein immobilization carrier disclosed in Patent Document 1, which is composed of zirconia (a biologically inert bioceramic) and a linker, has a problem in that the protein adsorbed thereon will tend to have a planar rigid surface and thus to denature and that its ability to bind to proteins is not enough.

The silica-based biomolecule carrier disclosed in Patent Document 2 is prepared by a process including: forming the linkers on the surface of the porous core; and then conjugating each of the first and second biologically active moieties to the linkers. The silica-based biomolecule carrier of Patent Document 2 has a problem in that the preparation process is complicated and that proteins adsorbed thereon are prone to denaturation because they are immobilized on the linkers and because the porous core is highly reactive due to a large number of silanol groups on the porous core. Thus, there is a need for a protein immobilization carrier that can be produced using a simpler method and is less likely to cause denaturation of proteins adsorbed thereon; a liquid dispersion of such a protein immobilization carrier; a hydrate of such a protein immobilization carrier; a protein-carrying complex including such a protein immobilization carrier; and an antibody test kit including such a protein immobilization carrier.

It is an object of the present invention, which has been made in view of those circumstances, to provide a protein immobilization carrier that can be produced using a simple method, is less likely to cause denaturation of proteins adsorbed thereon, and has a high ability to bind to proteins; a liquid dispersion of such a protein immobilization carrier; a hydrate of such a protein immobilization carrier; a protein-carrying complex including such a protein immobilization carrier; and an antibody test kit including such a protein immobilization carrier.

Means for Solving the Problems

To achieve the object, the present invention has the following technical features.

   (1) A protein immobilization carrier including an amorphous silica-based compound including silicon (Si), oxygen (O), and chlorine (Cl), the protein immobilization carrier having a solid-state $^{29}$Si-NMR spectrum with a peak area $Q_4$

derived from Si(OSi)$_4$, a peak area Q$_3$ derived from HO-Si(OSi)$_3$, and a peak area Q$_2$ derived from (HO)$_2$-Si(OSi)$_2$ and with a (Q$_2$ + Q$_3$)/(Q$_2$ + Q$_3$ + Q$_4$) ratio of 0.30 or less, the protein immobilization carrier having a chlorine atom molar concentration of 0.01 mol% or more as measured using X-ray fluorescence (XRF) analysis.

(2) The protein immobilization carrier according to aspect (1), wherein the protein immobilization carrier has a Fourier transform infrared (FT-IR) spectrophotometric spectrum with a ratio of a peak area of a peak at a wavenumber of 2800 cm$^{-1}$ to the integral of absorbance in the wavenumber range of 3800 cm$^{-1}$ to 2500 cm$^{-1}$ of 0.005 or more.

(3) The protein immobilization carrier according to aspect (1) or (2), wherein the protein immobilization carrier is in the form of particles with a minimum particle size of 50 nm or more.

(4) A protein immobilization carrier dispersion including: a dispersion medium including water; and the protein immobilization carrier according to any one of aspects (1) to (3) dispersed in the dispersion medium.

(5) The protein immobilization carrier dispersion according to aspect (4), wherein the dispersion medium includes a buffer solution.

(6) The protein immobilization carrier dispersion according to aspect (4) or (5), wherein the protein immobilization carrier has a surface zeta potential more positive than -20 mV.

(7) A protein immobilization carrier hydrate including: the protein immobilization carrier according to any one of aspects (1) to (3); and a hydrated layer on a surface of the protein immobilization carrier.

(8) The protein immobilization carrier hydrate according to aspect (7), wherein the protein immobilization carrier hydrate has a hydrated water weight concentration of 0.50% by weight or less based on the total weight of the carrier hydrate as determined using thermogravimetric-differential thermal analysis (TG-DTA).

(9) The protein immobilization carrier hydrate according to aspect (7) or (8),

wherein the protein immobilization carrier hydrate has a Fourier transform infrared (FT-IR) spectrophotometric spectrum with a first FT-IR peak at a wavenumber of 3190 ± 20 cm$^{-1}$ derived from asymmetric stretching vibration of O-H in water molecules, a second FT-IR peak at a wavenumber of 3260 ± 20 cm$^{-1}$ derived from deformation vibration of O-H in water molecules, a third FT-IR peak at a wavenumber of 3430 ± 20 cm$^{-1}$ derived from symmetric stretching vibration of O-H in water molecules, a fourth FT-IR peak at a wavenumber of 3480 ± 20 cm$^{-1}$ derived from stretching deformation vibration of water molecules on Si-OH, and a fifth FT-IR peak at a wavenumber of 3610 ± 20 cm$^{-1}$ derived from stretching vibration of O-H in hydrogen-bonded water molecules and from asymmetric stretching vibration of water molecules on Si-OH, wherein the first, second, third, fourth, and fifth FT-IR peaks are distinguishable from one another, and

wherein the Fourier transform infrared (FT-IR) spectrophotometric spectrum has a ratio of the area of the fourth FT-IR peak to the area of the first FT-IR peak of 0.25 or more.

(10) A protein-carrying complex including: the protein immobilization carrier hydrate according to any one of aspects (7) to (9); and a protein adsorbed on the hydrated layer of the protein immobilization carrier hydrate.

(11) An antibody test kit including a detection portion including at least the protein-carrying complex according to aspect (10), wherein the protein is an antibody protein.

Effects of the Invention

The present invention provides a protein immobilization carrier that can be produced using a simple method, is less likely to cause denaturation of proteins adsorbed thereon, and has a high ability to bind to proteins; a liquid dispersion of such a protein immobilization carrier; a hydrate of such a protein immobilization carrier; a protein-carrying complex including such a protein immobilization carrier; and an antibody test kit including such a protein immobilization carrier.

BRIEF DESCRIPTION OF THE DRAWINGS

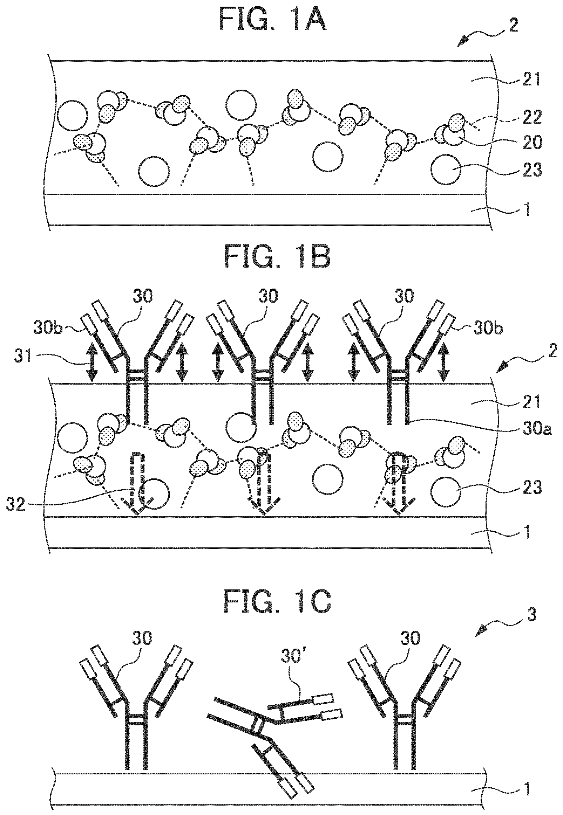

FIGS. 1A to 1C are schematic diagrams showing an example of the protein immobilization carrier hydrate of the present invention, an example of the protein-carrying complex of the present invention, and an example of a comparative protein-carrying complex, in which FIG. 1A shows an example of the protein immobilization carrier hydrate of the present invention, FIG. 1B shows an illustrative form in which an antibody protein is immobilized on the surface of the protein immobilization carrier hydrate of the present invention, and FIG. 1C shows, for comparison, an illustrative form in which an antibody protein is immobilized on the surface of a protein immobilization carrier free of chlorine;

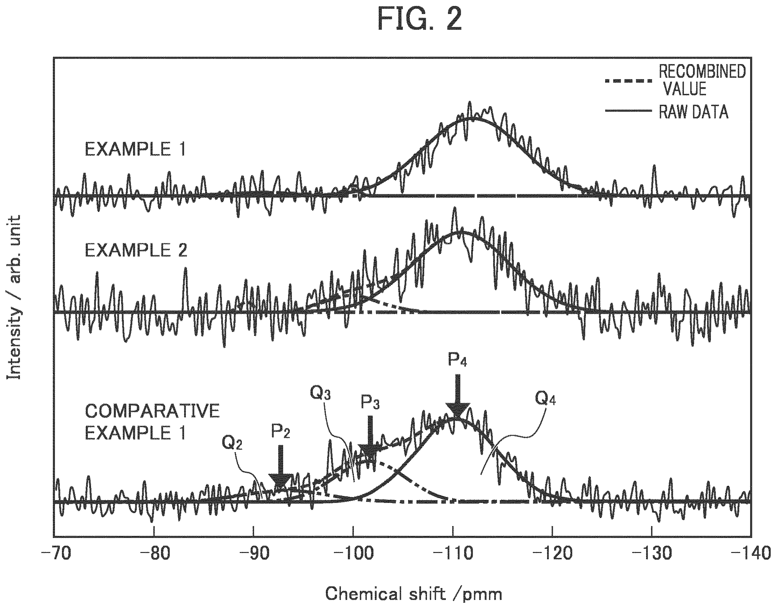

FIG. 2 shows solid-state $^{29}$Si-NMR spectra of the protein immobilization carriers of Examples 1 and 2 and Comparative Example 1;

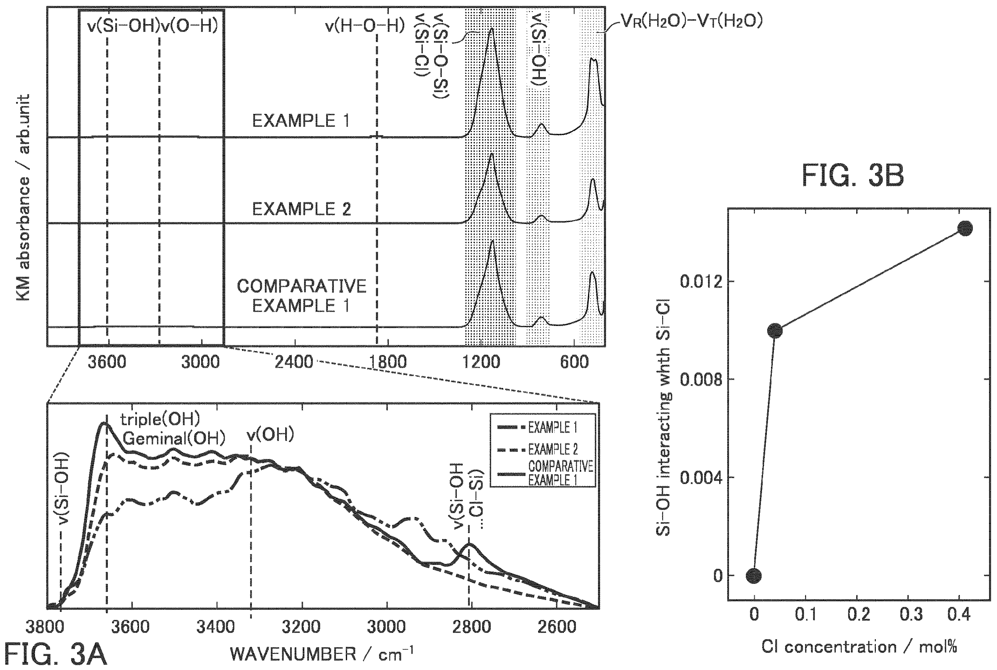

FIGS. 3A and 3B show graphs including: FIG. 3A showing FT-IR spectra measured of the protein immobilization carriers of Examples 1 and 2 and Comparative Example 1; and FIG. 3B showing, for the protein immobilization carriers of Examples 1 and 2 and Comparative Example 1, a graph that has a horizontal axis representing chlorine atom molar concentration and a vertical axis representing the peak area ratio relative to the peak area ratio of Comparative Example 1 (the reference value normalized to 0) and shows a relationship between the chlorine atom molar concentration and the peak area ratio;

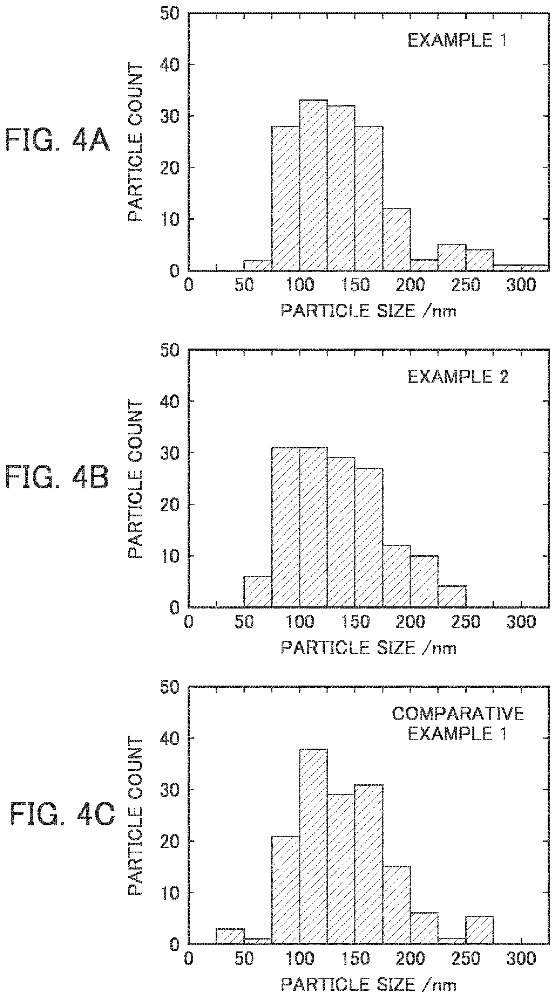

FIGS. 4A to 4C show graphs showing the particle size distribution of protein immobilization carriers, in which FIG. 4A shows the particle size distribution of the protein immobilization carrier of Example 1, FIG. 4B shows the particle size distribution of the protein immobilization carrier of Example 2, and FIG. 4C shows the particle size distribution of the protein immobilization carrier of Comparative Example 1;

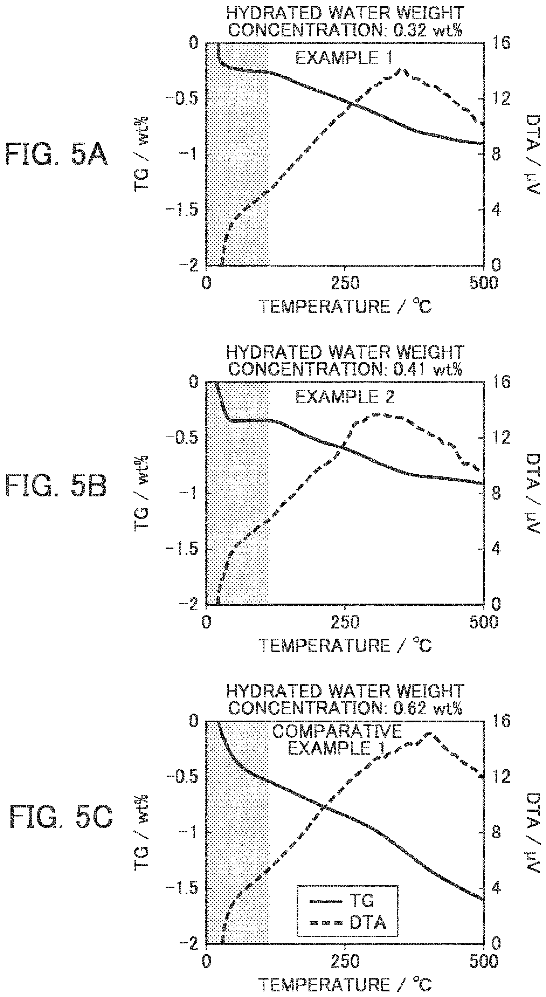

FIGS. 5A to 5C show TG-DTA charts with a horizontal axis representing temperature and a vertical axis representing thermal gravity (TG) and differential heat (DTA), in which FIG. 5A shows a TG-DTA chart of Example 1, FIG. 5B shows a TG-DTA chart of Example 2, and FIG. 5C shows a TG-DTA chart of Comparative Example 1;

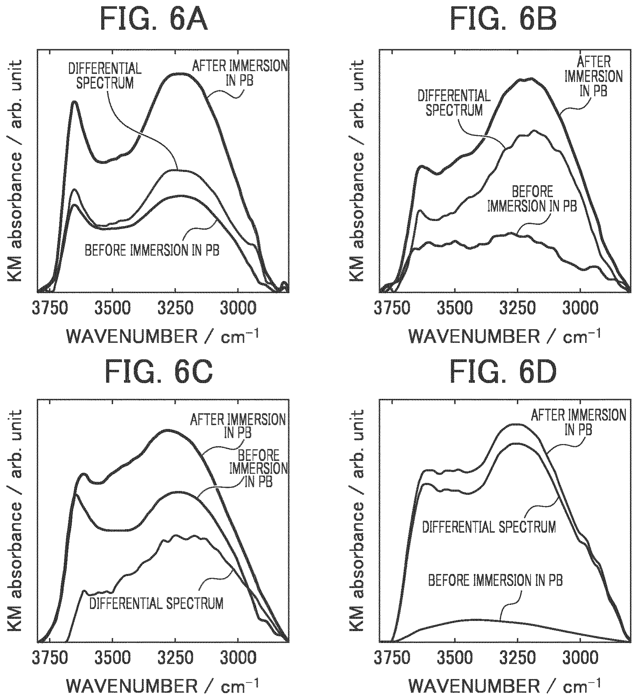

FIGS. 6A to 6D show charts showing an FT-IR spectrum of a protein immobilization carrier hydrate after immersion in a phosphoric acid buffer solution, an FT-IR spectrum of the protein immobilization carrier hydrate before immersion in the phosphoric acid buffer solution, and a differential spectrum between them, in which FIG. 6A, FIG. 6B, FIG. 6C, and FIG. 6D show charts of Example 1, Example 2, Comparative Example 1, and Comparative Example 2, respectively;

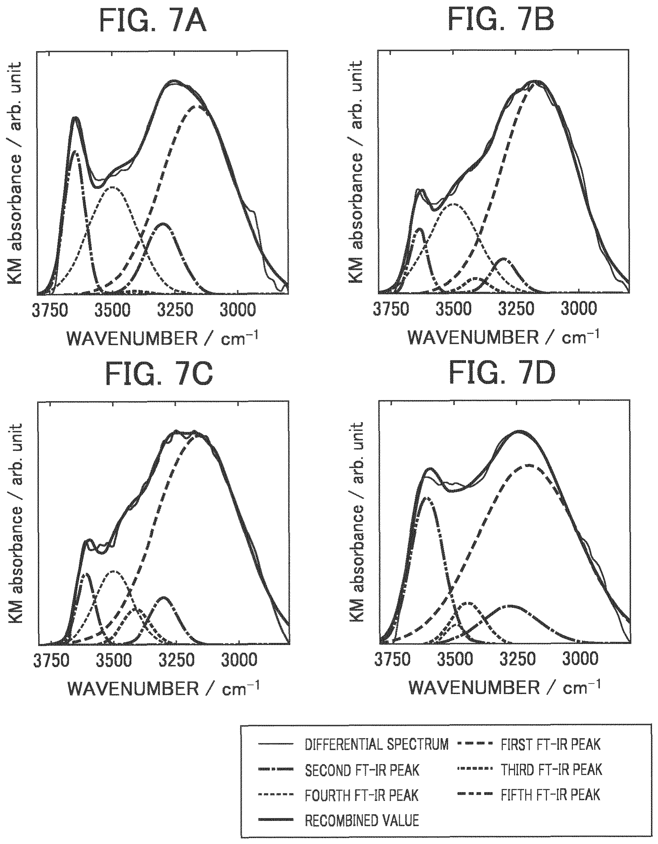

FIGS. 7A to 7D show charts showing FT-IR peaks separated from each other in the FT-IR spectra of FIG. 6A, FIG. 6b, FIG. 6C, and FIG. 6D, in which FIG. 7A, FIG. 7B, FIG. 7C, and FIG. 7D show charts of Example 1, Example 2, Comparative Example 1, and Comparative Example 2, respectively;

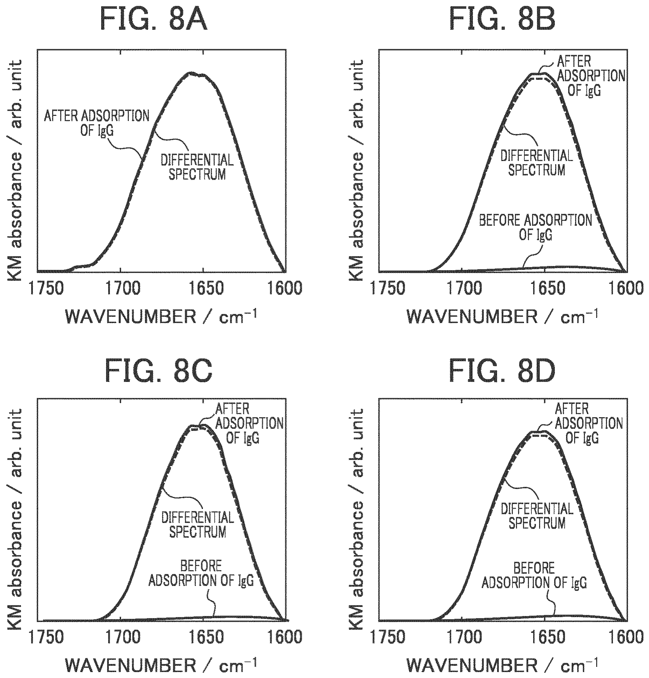

FIGS. 8A to 8D show charts showing an FT-IR spectrum of a protein-carrying complex after adsorption of IgG, an FT-IR spectrum of the protein immobilization carrier before adsorption of IgG, and a differential spectrum between them, in which FIG. 8A, FIG. 8B, FIG. 8C, and FIG. 8D show charts of Example 1, Example 2, Comparative Example 1, and Comparative Example 2, respectively; and

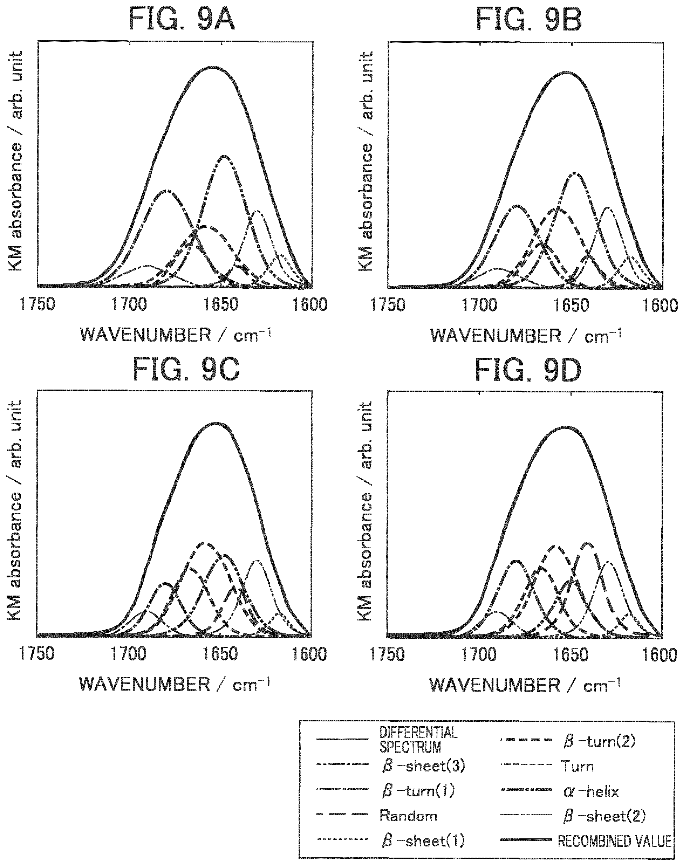

FIGS. 9A to 9D shows charts showing FT-IR peaks that are derived from portions of the IgG antibody and separated from each other in the FT-IR spectra of FIG. 8A, FIG. 8B, FIG. 8C, and FIG. 8D, in which parts FIG. 9A, FIG. 9B, FIG. 9C, and FIG. 9D show charts of Example 1, Example 2, Comparative Example 1, and Comparative Example 2, respectively.

## PREFERRED MODE FOR CARRYING OUT THE INVENTION

Hereinafter, specific embodiments of the present invention will be described in detail with reference to the drawings. It will be understood that the embodiments shown below are not intended to limit the present invention and may be altered or modified in various ways without departing from the gist of the present invention.

[1] Protein Immobilization Carrier

The protein immobilization carrier of the present invention includes an amorphous silica-based compound including silicon (Si), oxygen (0), and chlorine (Cl), has a solid-state $^{29}$Si-NMR spectrum with the area $Q_4$ of a peak $P_4$ derived from $Si(OSi)_4$, the area $Q_3$ of a peak $P_3$ derived from $HO\text{-}Si(OSi)_3$, and the area $Q_2$ of a peak $P_2$ derived from $(HO)_2\text{-}Si(OSi)_2$, has a $(Q_2 + Q_3)/(Q_2 + Q_3 + Q_4)$ ratio of 0.30 or less, and has a chlorine atom molar concentration of 0.01 mol% or more as measured using X-ray fluorescence (XRF) analysis.

The protein immobilization carrier of the present invention, which has a solid-state $^{29}$Si-NMR spectrum with a $(Q_2 + Q_3)/(Q_2 + Q_3 + Q_4)$ ratio of 0.30 or less, has a reduced content of silanol group (Si-OH)-forming silicon atoms in the silica-based compound, which forms the protein immobilization carrier. When dispersed in a dispersion medium including water, the protein immobilization carrier with such a reduced content of silanol group-forming silicon atoms can form a surface that is less likely to cause denaturation of proteins, such as antibody proteins, because only a reduced amount of water molecules can form hydrogen bonding to the oxygen atoms in the silanol groups so that alternatively, Si-O-Si bonds can be predominant.

The protein immobilization carrier of the present invention includes a chlorine (Cl)-containing amorphous silica-based compound. Thus, Si-Cl bonds having many unshared electron pairs can be included in the silica-based compound to form a highly interactive interface, which can increase the thickness of non-freezing water and intermediate water.

Thus, the protein immobilization carrier of the present invention can have a reduced thickness of free water and an increased thickness of non-freezing water and intermediate water, especially an increased thickness of intermediate water. When a protein is adsorbed on the protein immobilization carrier in an aqueous dispersion and then subjected to freeze drying, therefore, a highly hydrophilic moiety of the protein can adsorb on the surface of the protein immobilization carrier with a reduced distance between the surface of the protein immobilization carrier and the protein, which is due to a reduced thickness of free water. In addition, intermediate water can gently constrain the placement of the protein relative to the surface of the protein immobilization carrier, and intermediate water molecules for the protein immobilization carrier and the protein can repel to each other to prevent the contact between the surface of the protein immobilization carrier and the protein, which can keep a desired positional relationship between the protein and the surface of the protein immobilization carrier. Accordingly, the protein immobilization carrier of the present invention is less likely to cause denaturation of proteins adsorbed thereon and has a high ability to bind to proteins.

The protein immobilization carrier including an amorphous silica-based compound including silicon (Si), oxygen (O), and chlorine (Cl) and having a chlorine atom molar concentration of 0.01 mol% or more can also be produced by a simple method that includes reacting chlorine gas or chlorine compound gas such as silicon tetrachloride gas with a surface layer portion of a silica compound raw material including silicon (Si) and oxygen (O) to dope the silica compound with chloride (Cl).

Therefore, those features make it possible to provide a protein immobilization carrier that can be produced using a simple method, is less likely to cause denaturation of proteins adsorbed thereon, and has a high ability to bind to proteins; a liquid dispersion of such a protein immobilization carrier; a hydrate of such a protein immobilization carrier; a protein-carrying complex including such a protein immobilization carrier; and an antibody test kit including such a protein immobilization carrier.

As used herein, the term "non-freezing water" refers to water that forms a layer adjacent to the surface of the hydrated silica-based compound, is substantially fixed by forming hydrogen bonding to the siloxane bond (Si-O-Si) on the surface of the silica-based compound, and has the property of not freezing at a temperature lower than the melting point, which means that it is not removable by freeze-drying. As used herein, the term "intermediate water" refers to water that forms a layer adjacent to the outer side of the non-freezing water layer relative to the silica-based compound, is present between the non-freezing water and the free water, and has properties intermediate between those of the non-freezing water and the free water, which means that it is removable by freeze drying when it does not interact with the silica-based compound, but could remain and interact with the surface of the protein immobilization carrier hydrate or the surface of the protein-carrying complex after freeze drying. As used herein, the term "free water" refers to water that is present outside the intermediate water relative to the silica-based compound, can move freely, and is almost entirely removable by freeze drying.

In other words, non-freezing water, free water, and intermediate layer exist on the hydrated surface of the protein immobilization carrier, in which the free water freely moves around the hydrated surface, and the intermediate water exists between the non-freezing water and the free water and has properties between those of the non-freezing water and the free water. On the hydrated surface of the protein immobilization carrier, the non-freezing water, the intermediate water, and the free water exist in order of being closer to the hydrated surface. After these types of water are subjected to freeze drying, almost all of the non-freezing water, some of the intermediate water, and some of the free water will remain on the surface of the protein immobilization carrier.

The protein immobilization carrier of the present invention includes an amorphous silica-based compound including silicon (Si), oxygen (O), and chlorine (Cl) and containing chlorine atoms particularly in its surface layer portion. An amorphous silica-based compound has the property that, when dispersed in a dispersion medium including water, it can undergo hydrolysis at its siloxane bond (Si-O-Si) to produce a silanol group (Si-OH). Upon the hydrolysis, any chlorine (Cl), if present, in a surface layer portion of such a silica-based compound may form a Si-Cl bond. Such a Si-Cl bond in the surface layer portion of the silica-based compound may react with water in the air to form a silanol group (Si-OH). However, the protein immobilization carrier of the present invention can have Si-Cl bonds embedded in the silica-based compound treated with chlorine gas or chlorine compound gas such as silicon tetrachloride gas so that the surface of the silica-based compound will be less likely to produce silanol groups (Si-OH). When the protein immobilization carrier is dispersed in an aqueous dispersion medium, therefore, only a reduced amount of water molecules will directly or indirectly form hydrogen bonding to oxygen atoms of silanol groups. Instead, an increased amount of water molecules can directly or indirectly form hydrogen bonding to oxygen atoms of siloxane bonds to increase the thickness of non-freezing water.

The protein immobilization carrier has a solid-state $^{29}$Si-NMR spectrum with a $(Q_2 + Q_3)/(Q_2 + Q_3 + Q_4)$ ratio of 0.30 or less. In particular, the $(Q_2 + Q_3)/(Q_2 + Q_3 + Q_4)$ ratio is preferably 0.20 or less, more preferably 0.15 or less, even more preferably 0.07 or less. With such a small $(Q_2 + Q_3)/(Q_2 + Q_3 + Q_4)$ ratio, the silica-based compound in the protein immobilization carrier can have a reduced content of silicon atoms capable of forming silanol groups (Si-OH). Thus, the carrier will be less likely to cause denaturation of proteins immobilized thereon.

The protein immobilization carrier also has a chlorine atom molar concentration of 0.01 mol% or more as measured using X-ray fluorescence (XRF) analysis. In particular, the chlorine atom molar concentration is preferably 0.03 mol% or more, more preferably 0.20 mol% or more. With such an increased chlorine atom molar concentration, the protein immobilization carrier can have a surface layer portion containing Si-Cl bonds, which can be embedded in the silica-based compound when the protein immobilization carrier is dispersed in an aqueous dispersion medium, so that the surface of the silica-based compound will be less likely to produce silanol groups (Si-OH).

In particular, the protein immobilization carrier preferably has a Fourier transform infrared (FT-IR) spectrophotometric spectrum in which the ratio of a peak area of a peak at a wavenumber of 2800 cm$^{-1}$ to the integral of absorbance in the wavenumber range of 3800 cm$^{-1}$ to 2500 cm$^{-1}$ is 0.005 or more greater than that of a corresponding chlorine atom-free protein immobilization carrier. In the wavenumber range of 3800 cm$^{-1}$ to 2500 cm$^{-1}$, the spectrum will exhibit, at a wavenumber of around 2800 cm$^{-1}$, a peak caused by interaction between the Si-OH bond of a silanol group and the Si-Cl bond. Even when the silica-based compound is free of chlorine atoms, the absorbance of its spectrum will not reach 0 at a wavenumber of around 2800 cm$^{-1}$ due to the effect of the tails of other peaks in the wavenumber range of 2500 cm$^{-1}$ to 3800 cm$^{-1}$, which can be caused by structural relaxation due to water entry into the silica structure. Thus, the intensity of interaction between Si-OH bond and Si-Cl bond can be determined by calculating a relative peak area ratio by subtracting, from the ratio of the peak at a wavenumber of 2800 cm$^{-1}$ to the integral of absorbance in the wavenumber range of 3800 cm$^{-1}$ to 2500 cm$^{-1}$, the corresponding peak area ratio in the corresponding chlorine atom-free case. In particular, when the relative peak area ratio is 0.005 or more, Si-Cl bonds, which could otherwise easily react with water in the air to produce silanol groups, will be embedded in the silica-based compound, so that the protein immobilization carrier can have an effectively increased thickness of non-freezing water when dispersed in an aqueous dispersion medium. It should be noted that the silica-based compound in the protein immobilization carrier will necessarily have Si-OH bonds at least on its surface since water in the air can cause hydrolysis of its surface.

The silica-based compound for forming the protein immobilization carrier may be in the form of, for example, one or more selected from a particle, a bulk, a tubular rod, a substrate, a fiber, or a capillary. In particular, for immobilization of a larger amount of a protein per unit volume, the silica-based compound is preferably in the form of particles. Alternatively, the silica-based compound may be in the form of a bulk, a tubular rod, or a substrate, which has a planar or curved surface. In such a case, the planar or curved surface may have a micro flow channel(s).

The protein immobilization carrier is preferably in the form of particles with a minimum particle size of 50 nm or more. This feature ensures sufficient spaces for immobilizing proteins on the surface of the protein immobilization carrier so that proteins can be more stably immobilized on the protein immobilization carrier. In this regard, in a case where the protein immobilization carrier is to be administered to the human body or the non-human animal body, the minimum particle size of 50 nm or more will make it less toxic to the human or animal body. On the other hand, the protein immobilization carrier is preferably in the form of particles with a maximum particle size of 300 nm or less so that the protein immobilization carrier will be less likely to precipitate in a liquid dispersion.

The minimum particle size of the protein immobilization carrier can be measured using, for example, a field emission scanning electron microscope (FE-SEM). The minimum particle size of the protein immobilization carrier can be determined by a process including: measuring its particle size distribution using an FE-SEM; making, from the particle size distribution, a histogram showing the frequencies of occurrence (percentages (%) of the numbers) of particles in particle size classes at intervals of 25 nm, such as size classes of more than 0 nm to less than 25 nm, 25 nm to less than 50 nm, 50 nm to less than 75 nm, 75 nm to less than 100 nm, 100 nm to less than 125 nm, and so on; and defining the minimum particle size as the lower limit of the class in which the frequency reaches at least 5% for the first time from the smaller size side. For example, when the frequency reaches at least 5% for the first time in the size class of 50 nm to less than 75 nm, the minimum particle size may be determined to be 50 nm.

[2] Method for Producing the Protein Immobilization Carrier

The protein immobilization carrier of the present invention can be produced, for example, by reacting chlorine gas or chlorine compound gas such as silicon tetrachloride gas with a surface layer portion of a silica compound raw material including silicon (Si) and oxygen (O).

The silica compound raw material may be a material (such as a soot body or a by-product) produced during a conventionally known soot method for producing silica glass (e.g., VAD (Vapor-phase Axial Deposition) method for producing silica glass). More specifically, the silica compound raw material may include porous synthetic quartz glass materials of various shapes obtained through oxygen-hydrogen flame hydrolysis reaction in a silica glass manufacturing process that includes introducing silicon tetrachloride (raw material) into the central part of an oxygen-hydrogen flame burner and subjecting it to hydrolysis reaction in the oxygen-hydrogen flame to produce porous synthetic quartz glass (soot body) or may include particles that have escaped from the flame before they turn into porous synthetic quartz glass.

A surface layer portion of the silica compound raw material may be reacted with chlorine compound gas for chlorine doping, for example, by subjecting the silica compound raw material to heat treatment at about 800°C to about 1300°C for about 5 hours to about 48 hours in an atmosphere including inert gas and chlorine gas or chlorine compound gas such as silicon tetrachloride gas. The heat treatment temperature for the chlorine doping is preferably 900°C to 1200°C. The heat treatment time for the chlorine doping is preferably 10 hours to 40 hours.

[3] Protein Immobilization Carrier Dispersion

The protein immobilization carrier dispersion of the present invention (hereinafter also referred to as the "dispersion") includes a dispersion medium including water; and the protein immobilization carrier dispersed in the dispersion medium. More specifically, the dispersion of the present invention includes the protein immobilization carrier with a chlorine atom molar concentration of 0.01 mol% or more, which is dispersed in the dispersion medium including water. In such a case, the surface of the carrier can have a positive zeta potential or a negative zeta potential of which the absolute value is smaller than that of the surface of the corresponding protein immobilization carrier free of chlorine atoms. The surface of the protein immobilization carrier with such a zeta potential is less likely to cause denaturation of proteins immobilized thereon, which would otherwise occur due to a certain negative charge on a protein immobilization carrier surface.

In a case where the carrier surface has a positive zeta potential, cell-activating proteins (acidic proteins), such as albumin, can have a negative charge in a dispersion medium having a near-neutral pH (hydrogen-ion concentration). Thus, such cell-activating proteins can easily adsorb on the carrier surface in a biological liquid. On the other hand, in a case where the carrier surface has a negative zeta potential, antibody proteins (basic proteins) with an isoelectric point of around 8, such as immunoglobulins, can have a positive charge in a dispersion medium having a near-neutral pH (hydrogen-ion concentration). In particular, therefore, such antibody proteins can easily adsorb on the carrier surface.

The concentration of the protein immobilization carrier in the dispersion is typically, but not limited to, 0.1 mg/mL or more and 3 mg/mL or less.

The dispersion medium for the dispersion preferably includes a buffer solution. In such a case, proteins can be successfully immobilized on the protein immobilization carrier without being denatured in the dispersion.

The buffer solution for the dispersion medium may be one or more selected from the group consisting of a phosphoric acid buffer solution, an acetic acid buffer solution, an ammonium chloride buffer solution, a citric acid buffer solution, a carbonic acid buffer solution, and a Tris-hydrochloric acid buffer solution. Specifically, the phosphoric acid buffer solution (hereinafter also referred to as the "PB"), which contains phosphoric acid or a phosphate, can have a hydrogen-ion concentration (pH), for example, in the range of 5.2 or more and 8.3 or less. The acetic acid buffer solution, which contains acetic acid or an acetate, can have a hydrogen-ion concentration (pH), for example, in the range of 3.2 or more and 6.2 or less. The ammonium chloride buffer solution, which contains ammonium chloride, can have a hydrogen-ion concentration (pH), for example, in the range of 8.0 or more and 11.0 or less. The citric acid buffer solution, which contains citric acid or a citrate, can have a hydrogen-ion concentration (pH), for example, in the range of 7.1 or more and 8.9 or less. The carbonic acid buffer solution, which contains carbonic acid or a carbonate, can have a hydrogen-ion concentration (pH), for example, in the range of 9.2 or more and 10.6 or less. The Tris-hydrochloric acid buffer solution, which contains tris(hydroxymethyl)aminomethane or a hydrochloric acid salt thereof, can have a hydrogen-ion concentration (pH), for example, in the range of 7.1 or more and 8.9 or less.

The buffer solution for the dispersion medium may contain other non-buffer ions in addition to the buffer salt. For example, the phosphoric acid buffer solution may contain chloride ions as non-buffer ions. In such a case, the phosphoric acid buffer solution can have an increased ion concentration.

The ion concentration of the buffer solution for the dispersion medium, namely the total concentration of cations and anions, is typically, but not limited to, 0.5 mM or more and 15 mM or less.

In the protein immobilization carrier dispersion of the present invention, the protein immobilization carrier (dispersoid) preferably has a surface zeta potential more positive than -20 mV. In such a case, the carrier surface can have a negative zeta potential of which the absolute value is relatively small and thus can be less likely to cause protein denaturation, which would otherwise occur due to electrostatic interaction caused by a certain negative charge on the surface of the protein immobilization carrier. On the other hand, the protein immobilization carrier preferably has a negative surface zeta potential, more preferably a negative surface zeta potential more negative than -10 mV, so that the protein immobilization carrier can be less likely to precipitate in the dispersion.

The surface zeta potential of the protein immobilization carrier can be measured using, for example, a nanoparticle multi-analyzer (qNano (trade name) manufactured by Meiwafosis).

[4] Protein Immobilization Carrier Hydrate

The protein immobilization carrier hydrate of the present invention includes the protein immobilization carrier and a hydrated layer on the surface of the protein immobilization carrier. The hydrated layer, which is composed mainly of free water, preferably contains an appropriate amount of intermediate water and/or non-freezing water. The protein immobilization carrier hydrate with such features can be obtained, for example, by freeze-drying a dispersion of the protein immobilization carrier in a dispersion medium including water. The resulting protein immobilization carrier hydrate also preferably has the same properties as those of the protein immobilization carrier described above.

The protein immobilization carrier hydrate preferably has a hydrated water weight concentration of 0.50% by weight or less based on the total weight of the carrier hydrate as determined using thermogravimetric-differential thermal analysis (TG-DTA). With a low hydrated water weight concentration, the protein immobilization carrier hydrate can have a reduced thickness of free water in the hydrated layer. In such a case, an intermediate water layer formed on the surface of the protein immobilization carrier can repel another intermediate layer formed on the protein side, which can induce three-dimensionality for the protein conformation. As a result, the protein can be maintained in a desired positional relationship with respect to the surface of the protein immobilization carrier. This may result in much less denaturation of the protein adsorbed on the protein immobilization carrier hydrate.

The protein immobilization carrier hydrate also preferably has a Fourier transform infrared (FT-IR) spectrophotometric spectrum with a first FT-IR peak at a wavenumber of $3190 \pm 20$ cm$^{-1}$ derived from asymmetric stretching vibration of O-H in water molecules, a second FT-IR peak at a wavenumber of $3260 \pm 20$ cm$^{-1}$ derived from deformation vibration of O-H in water molecules, a third FT-IR peak at a wavenumber of $3430 \pm 20$ cm$^{-1}$ derived from symmetric stretching vibration of O-H in water molecules, a fourth FT-IR peak at a wavenumber of $3480 \pm 20$ cm$^{-1}$ derived from stretching deformation vibration of water molecules on Si-OH, and a fifth FT-IR peak at a wavenumber of $3610 \pm 20$ cm$^{-1}$ derived from stretching vibration of O-H in hydrogen-bonded water molecules and from asymmetric stretching vibration of water molecules on Si-OH, wherein the first, second, third, fourth, and fifth FT-IR peaks are distinguishable from one another, and the Fourier transform infrared (FT-IR) spectrophotometric spectrum preferably has a ratio of the area of the fourth FT-IR peak to the area of the first FT-IR peak of 0.25 or more, more preferably 0.30 or more. In this regard, free water molecules can contribute to the asymmetric stretching vibration of O-H in water molecules, represented by the first FT-IR peak, and to the deformation vibration of O-H in water molecules, represented by the second FT-IR peak. Intermediate water molecules can contribute to the symmetric stretching vibration of O-H in water molecules, represented by the third FT-IR peak, and to the stretching deformation vibration of water molecules on Si-OH, represented by the fourth FT-IR peak. Non-freezing water can contribute to the stretching vibration of O-H in hydrogen-bonded water molecules and to the asymmetric stretching vibration of water molecules on Si-OH, which is represented by the fifth FT-IR peak. Therefore, an increased ratio of the area of the fourth FT-IR peak to the area of the first FT-IR peak, such as 0.25 or more, will mean an increased amount of non-freezing water and intermediate water relative to the amount of free water. Thus, the protein immobilization carrier hydrate with such features can be less likely to cause denaturation of proteins adsorbed thereon.

The protein immobilization carrier hydrate may contain a non-water component in the hydrated layer. Examples of such a non-water component include buffer solution components, such as phosphate ions, sodium ions, and chloride ions.

[5] Protein-Carrying Complex

The protein-carrying complex of the present invention includes the protein immobilization carrier hydrate described above; and a protein adsorbed on the hydrated layer of the protein immobilization carrier hydrate. When the protein immobilization carrier hydrate is used, proteins adsorbed thereon will be less likely to make direct contact with the surface of the protein immobilization carrier and maintained in a desired positional relationship with respect to the surface of the protein immobilization carrier. In the protein-carrying complex of the present invention, therefore, the adsorbed protein will be less likely to denature.

FIGS. 1A to 1C are schematic diagrams showing an example of the protein immobilization carrier hydrate of the present invention, an example of the protein-carrying complex of the present invention, and an example of a comparative protein-carrying complex, in which FIG. 1A shows an example of the protein immobilization carrier hydrate of the present invention, FIG. 1B shows an illustrative form in which an antibody protein is immobilized on the surface of the protein immobilization carrier hydrate of the present invention, and FIG. 1C shows, for comparison, an illustrative form in which an antibody protein is immobilized on the surface of a protein immobilization carrier free of chlorine.

As shown in FIG. 1A, the protein immobilization carrier hydrate 2 includes a protein immobilization carrier 1 including a silica-based compound; and a hydrated layer 21 including water molecules 20 and provided on the surface of the protein immobilization carrier 1. Some of the water molecules 20 in the hydrated layer 21 form hydrogen bonding 22 with other water molecules 20 and the surface of the protein immobilization carrier 1 and thus form non-freezing water. The water molecules 20 in the hydrated layer 21 also include intermediate water molecules in addition to the non-freezing water molecules. The hydrated layer 21 may further contain buffer solution components 23 as non-water components.

As shown in FIG. 1B, the protein-carrying complex 3 includes the protein immobilization carrier hydrate 2; and protein molecules 30 adsorbed on the surface of the protein immobilization carrier 2. In the protein-carrying complex 3, the immobilized protein 30 preferably has at least a hydrophilic terminal 30a. In such a case, the hydrophilic terminal 30a of the protein 30 can be adsorbed on the surface of the protein immobilization carrier 1 and maintained inside the hydrated layer 21 so that the protein 30 will be less likely to denature.

In particular, the protein 30 more preferably has both a hydrophilic terminal 30a and a hydrophobic terminal 30b so that the hydrophilic terminal 30a of the protein 30 can be adsorbed on the hydrated surface of the protein immobilization carrier 1 and that the hydrophobic terminal 30b side, which is an intermediate water (not shown)-rich protein 30 side, can be located apart from the protein immobilization carrier 1 or the hydrated layer 21. In the protein-carrying complex 3 with such features, an intermediate water layer formed on the surface of the protein immobilization carrier 1, which forms the hydrate 2, can repel, as indicated by the double-headed arrows, another intermediate water layer formed on the hydrophobic terminal 30b side of the protein 30, so that the hydrophobic terminal 30b can be located apart from the protein immobilization carrier 1 or the hydrated layer 21. In the protein-carrying complex 3, the protein 30 adsorbed on the surface may also be an antibody protein or any other protein capable of binding to a target substance, such as an antigen (not shown). In such a case, the protein 30 immobilized on the protein immobilization carrier 1 preferably has a hydrophobic terminal 30b capable of binding to the target substance, so that the target substance can strongly bind to the protein 30.

On the other hand, if the protein immobilization carrier 1 is free of chlorine (Cl), the corresponding protein immobilization carrier hydrate 2 will have a reduced thickness of non-freezing water and intermediate water on the protein immobilization carrier 1 so that the hydrated layer 21 will fail to maintain the hydrophilic terminal 30a of the protein 30 in its interior. In addition, the intermediate water layer formed on the surface of the protein immobilization carrier 1 will be more distant from the intermediate water layer formed on the hydrophobic terminal 30b side of the protein 30 so that these layers will repel each other with a weaker force. This will result in random arrangement of the hydrophilic and hydrophobic terminals 30a and 30b of the protein 30 with respect to the surface of the protein immobilization carrier 1, which will result in contact between the surface of the protein immobilization carrier 1 and the protein 30. As a result, as shown in FIG. 1C, a denatured protein 30' will occur, which has at least a portion of the protein 30 denatured.

The protein immobilized in the protein-carrying complex of the present invention may have one or both of an $\alpha$-helix secondary structure and a $\beta$-sheet secondary structure and have the property of binding to a target substance such as an antigen. Proteins having a secondary structure can denature when at least a portion of the secondary structure is disordered and changed to a random or turn component. In particular, proteins capable of binding to target substances will tend to have difficulty in biding to the target substances when their molecular structure responsible for the binding to the target substances undergo such changes. In this regard, the protein-carrying complex of the present invention can maintain the protein in a desired positional relationship with respect to the surface of the protein immobilization carrier so that the target substance can specifically bind to the protein.

The protein adsorbed on the hydrated layer of the protein immobilization carrier hydrate can be prepared by dispersing the protein immobilization carrier in a dispersion including the protein and water to form a protein-containing hydrated layer. An alternative process may include dispersing the protein immobilization carrier in a dispersion medium including water to form the protein immobilization carrier hydrate; and then dispersing the protein immobilization carrier hydrate in a liquid dispersion containing at least the protein to adsorb the protein on the surface of the hydrated layer.

[6] Antibody Test Kit and Other Applications

In the protein-carrying complex of the present invention, the protein may be an antibody protein. In such a case, the protein-carrying complex may be used for an antibody test kit. Such an antibody test kit includes a detection portion including at least the antibody protein-carrying complex, in which the antibody protein is capable of binding to a target substance, such as an antigen, in a sample so that the target substance can be detected. Besides the above, the protein-carrying complex of the present invention may be used for materials requiring three-dimensional protein adsorption, more specifically for antithrombotic materials (biocompatible materials) such as catheters and other applications.

EXAMPLES

Hereinafter, the present invention will be described in more detail with reference to examples, which are not intended to limit the present invention.

Production of Protein Immobilization Carrier

A cylindrical porous soot body with an outer diameter of 300 mm and a length of 700 mm was formed by VAD (Vapor-phase Axial Deposition) using silicon tetrachloride as a raw material. The soot body was then crushed to give synthetic quartz glass particles (silica compound raw material). In this process for forming the porous soot body by VAD, silicon tetrachloride was introduced from a central portion of an oxygen-hydrogen flame burner, the flame zone with a temperature of at least 1000°C was 400 mm long, and the volume ratio of hydrogen to oxygen ($H_2/O_2$) was 1.5.

The resulting synthetic quartz glass particles were heated at 1100°C for 48 hours in a mixed atmosphere of inert gas ($N_2$) and silicon tetrachloride gas so that the synthetic quartz glass particles were doped with chlorine to produce a protein immobilization carrier of Example 1. Separately, the resulting synthetic quartz glass particles were heated at 1100°C for 48 hours in a mixed atmosphere of inert gas ($N_2$) and chlorine gas so that the synthetic quartz glass particles were doped with chlorine to produce a protein immobilization carrier of Example 2. Separately, the synthetic quartz glass particles not doped with chlorine were named a protein immobilization carrier of Comparative Example 1. In addition, commercially available synthetic quartz glass particles (product number: 7631-86-9 manufacture by Aerosil) were named a protein immobilization carrier of Comparative Example 2.

Methods for Measurement and Evaluation of Protein Immobilization Carrier

The properties of the protein immobilization carriers of Examples 1 and 2 and Comparative Example 1 were evaluated as shown below. The conditions for each property evaluation were as follows.

[1-1] Solid-State $^{29}$Si-NMR Spectrum of Protein Immobilization Carrier

The solid-state $^{29}$Si-NMR spectra of the protein immobilization carriers of Examples 1 and 2 and Comparative Example 1 were measured under the conditions below. FIG. 2 shows the resulting solid-state $^{29}$Si-NMR spectra.

Measurement Conditions

Instrument: Bruker Advance 300wbs Spectrometer (manufactured by Bruker)
Measurement method: DD (Dipolar Decoupling)-MAS (Magic Angle Spinning) method
Sample tube: 7 mm
Sample rotation speed: 5000 rpm
Resonance frequency: 59.62 MHz
Pulse width: 4.5 ms
Wait time: 60 sec
Number of scans: 1000 times
External standard sample: Hexamethylcyclotrisiloxane (-9.55 ppm)

The solid-state $^{29}$Si-NMR spectrum measurement process included pre preliminarily identifying the peak species using CP (Cross Polarization)-MAS method, which can detect Si near $^1$H with high sensitivity in short time; and then performing measurement using DD-MAS method. In this process, the CP-MAS method includes magnetically exciting highly sensitive hydrogen; and then performing Si-NMR measurement after magnetization transfer to Si, while the DD-MAS method includes directly exciting Si to give quantitative peaks.

The resulting solid-state $^{29}$Si-NMR spectrum was subjected to spectral separation for more detailed analysis. The solid-state $^{29}$Si-NMR spectral components can be assigned to three components for Si close to $^1$H. However, since these spectral components overlapped, the solid-state $^{29}$Si-NMR spectrum was separated into $Q_2$, $Q_3$, and $Q_4$ according to the peak separation method below.

Peak Separation Method
Baseline Creation Method
Step (1): Calculating average intensity value 1 in the range of -70 to -79 ppm and average intensity value 2 in the range of -131 to -140 ppm;
Step (2): Defining the baseline as the straight line passing through the two points (-70 ppm, average value 1) and (-140 ppm, average value 2);
Step (3): Removing the baseline value from the spectrum. Peak Separation Method
Separation method: Microsoft Office 2021 Excel (registered trademark) Solver function
Function used: Gaussian function below (where A is the peak height, B is the peak position, and C is the half-width.)

$$f(x) = A\exp\left\{-\frac{(x-B)^2}{C^2}\right\}$$

Peak assignment:

$Q_2$: -93 $\pm$ 2 ppm for two $\equiv$Si-O-Si$\equiv$ bonds and two $\equiv$Si-OH bonds
$Q_3$: -102 $\pm$ 2 ppm for three $\equiv$Si-O-Si$\equiv$ bonds and one $\equiv$Si-OH bond
$Q_4$: -111 $\pm$ 2 ppm for four $\equiv$Si-O-Si bonds
Initial conditions for A, B, and C:
$Q_2$: A = 100000, B = -93, C = 4
$Q_3$: A = 400000, B = -102, C = 5.5
$Q_4$: A = 960000, B = -111, C = 5.5

The resulting solid-state $^{29}$Si-NMR spectra of the protein immobilization carriers of Examples 1 and 2 and Comparative Example 1 shown in FIG. 2 were named raw data and subjected to spectral separation as shown above. For each of the solid-state $^{29}$Si-NMR spectra, the $(Q_2 + Q_3)/(Q_2 + Q_3 + Q_4)$ ratio was calculated, in which $Q_4$ is the calculated peak area derived from $Si(OSi)_4$, $Q_3$ is the calculated peak area derived from $HO\text{-}Si(OSi)_3$, and $Q_2$ is the calculated peak area derived from $(HO)_2\text{-}Si(OSi)_2$. The results are shown in Table 1.

[Table 1]

|  | $Q_2$ | $Q_3$ | $Q_4$ | $(Q_2+Q_3)/(Q_2+Q_3+Q_4)$ |
|---|---|---|---|---|
| Example 1 | 2.8 | 1.0 | 96.2 | 0.038 |
| Example 2 | 1.7 | 10.1 | 88.2 | 0.118 |
| Comparative Example 1 | 8.5 | 26.7 | 64.8 | **<u>0.352</u>** |
| (Note) Bold and underline indicate that the value is outside the scope of the present invention. | | | | |

As a result, the protein immobilization carriers of Examples 1 and 2 each had a $(Q_2 + Q_3)/(Q_2 + Q_3 + Q_4)$ ratio of at most 0.30, which falls within the scope of the present invention. On the other hand, the protein immobilization carrier of Comparative Example 1 had a $(Q_2 + Q_3)/(Q_2 + Q_3 + Q_4)$ ratio of more than 0.30, which is outside the scope of the present invention.

[1-2] Chemical Composition Measured Using X-ray Fluorescence (XRF) Analysis of Protein Immobilization Carrier

A powder sample (100 mg) of each of the protein immobilization carriers of Examples 1 and 2 and Comparative Example 1 was compressed into a pellet without being diluted. The resulting sample pellet was subjected to XRF analysis using an X-ray fluorescence analyzer (ZSX Primus II (model name) manufactured by Rigaku) and the accompanying semi-quantitative analysis software (EZ Scan Program (software name) manufactured by Rigaku). During the measurement, semi-quantitative analysis was performed using the measurement program supplied with the analyzer while no primary filter or calibration curve was used. The XRF analysis was performed three times for one sample pellet, and the average of three measurements of the silicon (Si), oxygen (O), and chlorine (Cl) contents was used as the analysis result. Table 2 shows the results of analysis of the samples of Examples 1 and 2 and Comparative Example 1.

[Table 2]

|  | O(mol%) | Si(mol%) | Cl(mol%) |
|---|---|---|---|
| Example 1 | 67.6 | 32.0 | 0.40 |
| Example 2 | 67.3 | 32.6 | 0.05 |
| Comparative Example 1 | 67.7 | 32.3 | **<u>0</u>** |
| (Note) Bold and underline indicate that the value is outside the scope of the present invention. | | | |

As a result, the protein immobilization carriers of Examples 1 and 2 each had a chlorine atom molar concentration of at least 0.01 mol%, which falls within the scope of the present invention. On the other hand, the protein immobilization carrier of Comparative Example 1 had a chlorine atom molar concentration of less than 0.01 mol%, which is outside the scope of the present invention.

[1-3] Fourier Transform Infrared (FT-IR) Spectrophotometric Analysis of Protein Immobilization Carrier

The protein immobilization carriers of Examples 1 and 2 and Comparative Example 1 were analyzed by diffusion reflection method using a Fourier transform infrared spectrophotometer (FT-IR) (FT/IR-4600 (product name) manufactured by JASCO) and KBr (background) under the conditions below. More specifically, 49 mg of KBr and 1 mg of the protein immobilization carrier were mixed to form a sample, which was subjected to the analysis under the conditions of the wavenumber range of 4000 cm$^{-1}$ to 400 cm$^{-1}$, a number of scans of 128, and a spectroscopic resolution of 4 cm$^{-1}$. The measurement result was subjected to subtraction of the KBr background spectrum and then to KM (Kubelka-Munk) transformation, resulting in an FT-IR spectrum showing the intensity ratio proportional to the concentration. Part (a) of FIGS. 3A and 3B show the FT-IR spectra obtained for the protein immobilization carriers of Examples 1 and 2 and Comparative Example 1.

From each of the FT-IR spectra in FIG. 3A, the integral of absorbance in the wavenumber range of 3800 $cm^{-1}$ to 2500 $cm^{-1}$ and the peak area of a peak at a wavenumber of 2800 $cm^{-1}$ were each calculated using the software (Spectra Manager manufactured by JASCO) supplied with the FT-IR, and the ratio (peak area ratio) of the peak area of a peak at a wavenumber of 2800 $cm^{-1}$ to the integral of absorbance in the wavenumber range of 3800 $cm^{-1}$ to 2500 $cm^{-1}$ was calculated. The peak area of a peak at a wavenumber of 2800 $cm^{-1}$ was the integral of peak region absorbance in the wavenumber range of 2825 $cm^{-1}$ to 2764 $cm^{-1}$, in which the peak region absorbance was the relative value calculated relative to the height (normalized to 0 (reference value)) of a baseline obtained by connecting a vertically lowest point in the wavenumber range of 2900 $cm^{-1}$ to 2800 $cm^{-1}$ and another vertically lowest point in the wavenumber range of 3800 $cm^{-1}$ to 3700 $cm^{-1}$. FIG. 3B is a graph showing a relationship between the chlorine atom molar concentration and the peak area ratio for the protein immobilization carriers of Examples 1 and 2 and Comparative Example 1 with the horizontal axis representing the chlorine atom molar concentration and the vertical axis representing the peak area ratio relative to that of Comparative Example 1, which is normalized to 0 (reference value).

A relative peak area ratio was calculated by subtracting the peak area ratio of Comparative Example 1 (free of chlorine atoms) from the peak area ratio of each of Examples 1 and 2 and Comparative Example 1 in order to eliminate the effect of the tails of other peaks, which can be caused by structural relaxation due to water entry into the silica structure. The results are shown in Table 3.

[Table 3]

|  | Integral (A) of absorbance in the wavenumber range 3800 $cm^{-1}$ to 2500 $cm^{-1}$ | Peak area (B) at wavenumber 2800 $cm^{-1}$ | Peak area ratio (B/A) | Relative peak area ratio calculated by subtracting peak area ratio of Comparative Example 1 from peak area ratio (B/A) |
|---|---|---|---|---|
| Example 1 | 17.2 | 0.51 | 0.029 | 0.014 |
| Example 2 | 6.30 | 0.16 | 0.025 | 0.010 |
| Comparative Example 1 | 15.6 | 0.24 | 0.015 | **0** |
| (Note) Bold and underline indicate that the value is outside a preferred range of the present invention. | | | | |

As a result, the peak area ratio of the protein immobilization carrier of each of Examples 1 and 2, which is the ratio of the peak area of a peak at the wavenumber of 2800 $cm^{-1}$ to the integral of absorbance in the wavenumber range of 3800 $cm^{-1}$ to 2500 $cm^{-1}$, was at least 0.005 greater than that of Comparative Example 1 free of chlorine atoms. Since the peak can be caused by the interaction between silanol Si-OH bond and Si-Cl bond, this suggests that the protein immobilization carrier of Example 1 or 2 should contain chlorine atoms at least in its surface layer portion.

[1-4] Measurement of Minimum Particle Size of Protein Immobilization Carrier

The minimum particle size of the protein immobilization carrier of each of Examples 1 and 2 and Comparative Example 1 was measured using a field emission scanning electron microscope (FE-SEM) (SU-8230 (product name) manufactured by Hitachi). More specifically, the protein immobilization carrier was fixed with carbon black to form a sample, which was subjected, under vacuum, to FE-SEM imaging at an acceleration voltage of 200 kV with a magnification of 100000× and a field of view of 1.62 μm (vertical) × 2.16 μm (horizontal). From the resulting FE-SEM image, 75 particles of the protein immobilization carrier were randomly selected and counted, and their sizes in the vertical and horizontal directions were each measured using the scale bar of the FE-SEM image. The average of the 150 numerical values resulting from the size measurement of the 75 carrier particles in the vertical and horizontal directions was determined to be the average particle size of each carrier. In addition, the particle size distribution was obtained from the measured particle sizes of each carrier and then used to make a histogram showing the frequencies of occurrence (percentages (%) of the numbers) of particles in particle size classes at intervals of 25 nm, such as size classes of more than 0 nm to less than 25 nm, 25 nm to less than 50 nm, 50 nm to less than 75 nm, 75 nm to less than 100 nm, 100 nm to less than 125 nm, and so on. Using the histogram, the minimum particle size was defined as the lower limit of the class in which the frequency reached at least 5% for the first time from the smaller size side. FIG. 4A, FIG. 4B, and FIG. 4C show the particle size distributions of the protein immobilization carriers in the protein immobilization carrier dispersions of Examples 1 and 2 and Comparative Example 1, respectively. Table 4 shows the resulting minimum value of the measured particle sizes.

[Table 4]

|  | Minimum particle size [nm] of protein immobilization carrier |
| --- | --- |
| Example 1 | 100 |
| Example 2 | 100 |
| Comparative Example 1 | 75 |
| (Note) Bold and underline indicate that the value is outside a preferred range of the present invention. | |

As a result, the protein immobilization carriers of Examples 1 and 2 and Comparative Example 1 each had a minimum particle size of at least 50 nm, which falls within a preferred range of the present invention. Specifically, the protein immobilization carrier of Example 1 or 2 had a minimum particle size as large as at least 100 nm, which may be due to the bonding between multiple particles during the chlorine doping.

Production of Protein Immobilization Carrier Dispersion

The protein immobilization carrier of Example 1 was dried at a temperature of 120°C for 12 hours, and then 10 mg of the dried protein immobilization carrier was mixed with a 10 mL of a sterilized 1 mM phosphoric acid buffer solution. The mixture was subjected to three times of mixing operation including application of ultrasonic vibration (frequency 40 kHz, power 360 W) for 5 minutes followed by shaking (vortexing) for 10 seconds. The resulting mixture was then agitated at a liquid temperature of 37°C for 6 hours to give a protein immobilization carrier dispersion of Example 1. The protein immobilization carriers of Example 2 and Comparative Example 1 were also subjected to the mixing with the 1 mM phosphoric acid buffer solution, the mixing operation, and the agitation to form protein immobilization carrier dispersions of Example 2 and Comparative Example 1.

Methods for Measurement and Evaluation of Protein Immobilization Carrier Dispersion

The properties of the protein immobilization carrier dispersions of Examples 1 and 2 and Comparative Example 1 were evaluated as shown below. The conditions for each property evaluation were as follows.

[2-1] Measurement of the Zeta Potential of Protein Immobilization Carrier in Dispersion

The surface zeta potential of the protein immobilization carrier in the protein immobilization carrier dispersion was measured by electrical resistance nano-pulse method using a nanoparticle multi-analyzer (qNano (trade name) manufactured by Meiwafosis).
More specifically, the protein immobilization carrier dispersion was mixed with a 100 mM phosphate buffered saline (PBS buffer solution with a pH of 7.4) containing 0.01% by volume of 4-(1,1,3,3-tetramethylbutyl)phenyl-polyethylene glycol (Triton-X-100 manufactured by Sigma-Aldrich) and 3 mM of ethylenediaminetetraacetic acid (EDTA). The mixture was subjected to ultrasonic dispersion for 10 minutes to give a dispersion of 50 ng/mL of the protein immobilization carrier. A 40 $\mu$L aliquot of the protein immobilization carrier dispersion was filled into the upper half of the liquid cell of qNano, while an 80 $\mu$L aliquot was filled into the lower half of the liquid cell. Next, an ion current was applied to the liquid cell, and the resulting current value (current pulse signal) was recorded. The current pulse signal was converted into digital data at a frequency of 1 MHz using an analog-to-digital converter, and the digital data was analyzed after being reduced to a sampling rate of 50 kHz by electronic filtering. The digital data was analyzed using IZON proprietary software (ver. 2.4), and data for at least 100 carrier particles were recorded per each measurement condition. A voltage of 0.70 V was applied to provide a current value of about 100 nA during the measurement. A calibration sample was also prepared by dispersing carboxylated polystyrene standard particles (calibration particles) with known particle sizes manufactured by Izon Science (CPC100 with an average particle size 100 nm) in a 100 mM PBS buffer solution containing 0.01% by volume of 4-(1,1,3,3-tetramethylbutyl)phenyl-polyethylene glycol and 3 mM of EDTA. The particle size of the calibration sample was evaluated and used for calibration when the particle size of the carrier was calculated.
The relationship between the electrophoretic velocity $v_x$ and zeta potential of the particle x being measured can be represented by Smoluchowski equation (a) below.

$$(v_x)_{el\ Sample} / (v_x)_{el\ Cal} = \zeta_{net\ sample} / \zeta_{net\ cal} \qquad (a)$$

In the Smoluchowski equation (a), $(v_x)_{el\ Sample}$ and $(v_x)_{el\ Cal}$ represent the electrophoretic velocities of the carrier particle and the calibration particle, respectively. In the equation (a), $\zeta_{net\ sample}$ and $\zeta_{net\ cal}$ represent the net zeta potentials of the sample particle and the calibration particle, respectively. The net zeta potential is the difference between the zeta potential ($\zeta_{P\,sample}$) of each particle at any applied pressure P and the zeta potential ($\zeta_m$) of the membrane as shown in equation (b).

$$\zeta_{net\ sample} = \zeta_{P\ sample} - \zeta_m \qquad (b)$$

The zeta potential ($\zeta^i_{sample}$) of each sample particle i can be given by averaging the zeta potential values calculated at various locations $l_x$ within the pores, and $l_x$ represents the location within the pores, at which the particle arrives after a certain time. The zeta potential was evaluated by taking the average of $l_x$ at several discrete points as shown by equation (c) below.

$$\xi^i_{Sample} = \frac{\sum_x \xi^i_{x\,Sample}}{\sum_x} = \frac{\sum_x (v^i_{x\,Sample} - v^P_{x\,cal}P)/(v^V_{x\,cal}V)}{\sum_x}\xi_{net\ Cal} + \xi_m \qquad \cdot\cdot\cdot (c)$$

In equation (c), $v^P_{x\,cal}$ is the convective velocity per unit pressure, $v^V_{x\,cal}$ is the interfacial electrokinetic velocity per unit voltage, P is the applied pressure, and V is the voltage during measurement. P and V are parameters at the time of particle size measurement, and $v^P_{x\,cal}$ and $v^V_{x\,cal}$ are determined at the time of particle size measurement. In this way, the zeta potential was measured through the measurement of the carrier particles-containing sample and the calibration sample. The resulting measured zeta potentials are shown in Table 5.

[Table 5]

| | Surface zeta potential [mV] of protein immobilization carrier |
|---|---|
| Example 1 | -17 |
| Example 2 | -17 |
| Comparative Example 1 | **<u>-32</u>** |
| (Note) Bold and underline indicate that the value is outside a preferred range of the present invention. | |

The resulting surface zeta potentials of the protein immobilization carriers of Examples 1 and 2 were found to be more positive than -20 mV, which falls within a preferred range of the present invention. On the other hand, the surface zeta potential of the protein immobilization carrier of Comparative Example 1 was found to be more negative than -20 mV, which is outside a preferred range of the present invention.

[2-2] Chemical Composition of Protein Immobilization Carrier in Dispersion Measured by X-ray Fluorescence (XRF) Analysis

After the preparation, the protein immobilization carrier dispersions of Examples 1 and 2 and Comparative Example 1 were allowed to stand for 48 hours so that the particles were allowed to precipitate. The resulting reduced-turbidity fraction of each dispersion was placed in a centrifuge tube and centrifuged at a liquid temperature of 4°C and a centrifugal force of 13000 × g for 5 minutes using a centrifuge (3700 (model number) manufactured by Kubota) so that the dispersoid was separated. After being washed with ultra-pure water, the resulting dispersoid was subjected again to centrifugation at a liquid temperature of 4°C and a centrifugal force of 13000 × g for 5 minutes so that the dispersoid was separated. The centrifuge tube containing the dispersoid was capped and then subjected to freeze drying under the conditions of -50°C and 3.0 Pa for 12 hours using a freeze dryer (FDU-1200 (model number) manufactured by Tokyo Rikakikai) so that a freeze-dried sample was obtained. The resulting sample was compressed at a force of 300 kPa for 2 minutes using oil hydraulic press to form a sample pellet. The sample pellet was subjected to XRF analysis using an X-ray fluorescence analyzer (ZSX Primus II (model name) manufactured by Rigaku) and the accompanying semi-quantitative analysis software (EZ Scan Program (software name) manufactured by Rigaku). During the measurement, semi-quantitative analysis was performed using the measurement program supplied with the analyzer while no primary filter or calibration curve was used. The XRF analysis was performed three times for one sample pellet, and the average of three measurements of the phosphorus (P) and sodium (Na) contents was used as the analysis result. Table 6 shows the analysis results for Examples 1 and 2 and Comparative Example 1.

**EP 4 671 764 A1**

[Table 6]

|  | P (mol%) | Na (mol%) |
|---|---|---|
| Example 1 | 0.80 | 1.50 |
| Example 2 | 0.40 | 0.80 |
| Comparative Example 1 | 0.04 | 0.00 |

As a result, the protein immobilization carrier in the dispersion of each of Examples 1 and 2 had a phosphorus (P) content of at least 0.40 mol% and a sodium (Na) content of at least 0.40 mol%, which suggests the incorporation of phosphorus (P) and sodium (Na) into its surface. On the other hand, the protein immobilization carrier in the dispersion of Comparative Example 1 contained almost no phosphorus (P) or sodium (Na), which suggests almost no incorporation of phosphorus (P) or sodium (Na) into its surface.

Production of Protein Immobilization Carrier Hydrate

The protein immobilization carrier of Example 1 was dried at a temperature of 120°C for 12 hours, and then 10 mg of the dried protein immobilization carrier was mixed with 10 mL of a sterilized 1 mM phosphoric acid buffer solution. The mixture was subjected to three times of mixing operation including application of ultrasonic vibration (frequency 40 kHz, power 360 W) for 5 minutes followed by shaking (vortexing) for 10 seconds. The resulting mixture was then agitated at a liquid temperature of 37°C for 6 hours to give a protein immobilization carrier dispersion. The protein immobilization carrier dispersion was placed in a centrifuge tube and centrifuged at a liquid temperature of 4°C and a centrifugal force of 13000 × g for 30 minutes using a centrifuge (3700 (model number) manufactured by Kubota) so that the dispersoid was separated. After being washed with ultra-pure water, the resulting dispersoid was subjected again to centrifugation at a liquid temperature of 4°C and a centrifugal force of 13000 × g for 30 minutes so that the dispersoid was separated. The centrifuge tube containing the dispersoid was capped and then subjected to freeze drying under the conditions of -50°C and 3.0 Pa for 12 hours using a freeze dryer (FDU-1200 (model number) manufactured by Tokyo Rikakikai) so that a protein immobilization carrier hydrate of Example 1 was obtained. The protein immobilization carriers of Example 2 and Comparative Examples 1 and 2 were also subjected to the same procedure to produce protein immobilization carrier hydrates of Example 2 and Comparative Examples 1 and 2.

[3-1] Hydrated Water Weight Concentration of Protein Immobilization Carrier Hydrate

The protein immobilization carrier hydrates of Examples 1 and 2 and Comparative Example 1 were measured for hydrated water weight concentration using a thermogravimetric-differential thermal analyzer (TG-8120 (model number) manufactured by Rigaku). More specifically, 10 mg to 20 mg of the protein immobilization carrier hydrate was placed on a sample stage and heated at a rate of 10°C/min from room temperature to 500°C in the air atmosphere when a TG-DTA chart with temperature on the horizontal axis and thermal gravity (TG) and differential heat (DTA) on the vertical axis was obtained. The TG-DTA charts for the protein immobilization carrier hydrates of Examples 1 and 2 and Comparative Example 1 are shown in FIG. 5A, FIG. 5B, and FIG. 5C, respectively. From each of the TG-DTA charts, the mass loss during heating from room temperature to 150°C was determined to be the hydrated water weight concentration. The results are shown in Table 7.

[Table 7]

|  | Hydrated water weight concentration [mass %] |
|---|---|
| Example 1 | 0.32 |
| Example 2 | 0.41 |
| Comparative Example 1 | **0.62** |
| (Note) Bold and underline indicate that the value is outside a preferred range of the present invention. ||

As a result, as determined by thermogravimetric-differential thermal analysis (TG-DTA), the protein immobilization carrier hydrates of Examples 1 and 2 each had a hydrated water weight concentration of at most 0.50% by weight based on the total weight of the carrier hydrate, which falls within a preferred range. On the other hand, as determined by thermogravimetric-differential thermal analysis (TG-DTA), the protein immobilization carrier hydrate of Comparative Example 1 had a hydrated water weight concentration of 0.62% by weight based on the total weight of the carrier hydrate, which is outside a preferred range.

[3-2] Fourier Transform Infrared (FT-IR) Spectrophotometric Analysis of Protein Immobilization Carrier Hydrate

The protein immobilization carrier hydrates of Examples 1 and 2 and Comparative Examples 1 and 2 were analyzed by diffusion reflection method using a Fourier transform infrared spectrophotometer (FT-IR) (FT/IR-4600 (product name) manufactured by JASCO) and KBr (background) under the conditions below. More specifically, 49 mg of KBr and 1 mg of the protein immobilization carrier hydrate were mixed to form a sample, which was subjected to the analysis under the conditions of the wavenumber range of 3800 cm$^{-1}$ to 2800 cm$^{-1}$, a number of scans of 128, and a spectroscopic resolution of 4 cm$^{-1}$. The measurement result was subjected to subtraction of the KBr background spectrum and then to KM (Kubelka-Munk) transformation, resulting in an FT-IR spectrum showing the intensity ratio proportional to the concentration.

In order to eliminate the effect of vibration (at a wavenumber of 3750 cm$^{-1}$) derived from Si-OH bond in amorphous silica, the resulting FT-IR spectrum (after immersion in PB) was overlaid on the FT-IR spectrum (before immersion in PB) obtained for [1-3] Fourier Transform Infrared (FT-IR) Spectrophotometric Analysis of Protein Immobilization Carrier, and the difference between them was calculated to create an FT-IR spectrum (differential spectrum) for the vibration of water molecules provided by hydration. The resulting FT-IR spectra for Examples 1 and 2 and Comparative Examples 1 and 2 are shown in FIG. 6A, FIG. 6B, FIG. 6C, and FIG. 6D, respectively.

Using the pseudo-Voigt function, the FT-IR spectrum in each of FIG. 6A, FIG. 6B, FIG. 6C, and FIG. 6D was subjected to peak separation into: a first FT-IR peak at a wavenumber of 3190 $\pm$ 20 cm$^{-1}$ derived from asymmetric stretching vibration of O-H in water molecules; a second FT-IR peak at a wavenumber of 3260 $\pm$ 20 cm$^{-1}$ derived from deformation vibration of O-H in water molecules; a third FT-IR peak at a wavenumber of 3430 $\pm$ 20 cm$^{-1}$ derived from symmetric stretching vibration of O-H in water molecules; a fourth FT-IR peak at a wavenumber of 3480 $\pm$ 20 cm$^{-1}$ derived from stretching deformation vibration of water molecules on Si-OH; and a fifth FT-IR peak at a wavenumber of 3610 $\pm$ 20 cm$^{-1}$ derived from stretching vibration of O-H in hydrogen-bonded water molecules and from asymmetric stretching vibration of water molecules on Si-OH. The area of each of the FT-IR peaks was calculated, and the ratio of the area of the fourth FT-IR peak to the area of the first FT-IR peak was calculated. The results are shown in Table 8. Peaks separated from the FT-IR spectra in FIG. 6A, FIG. 6b, FIG. 6C, and FIG. 6D are shown in FIG. 7A, FIG. 7B, FIG. 7C, and FIG. 7D, respectively.

[Table 8]

| | Area (W) of first FT-IR peak | Area (X) of second FT-IR peak | Area (Y) of third FT-IR peak | Area (Z) of fourth FT-IR peak | Ratio (Z/W) of fourth FT-IR peak area to first FT-IR peak area |
|---|---|---|---|---|---|
| Example 1 | 56.391 | 10.007 | 0.2903 | 21.529 | 0.38 |
| Example 2 | 69.208 | 3.9225 | 1.5821 | 20.438 | 0.30 |
| Comparative Example 1 | 73.166 | 5.4817 | 4.1094 | 11.794 | **0.16** |
| Comparative Example 2 | 66.721 | 8.5569 | 5.1566 | 1.4588 | **0.02** |
| (Note) Bold and underline indicate that the value is outside a preferred range of the present invention. | | | | | |

As a result, the protein immobilization carrier hydrates of Examples 1 and 2 each had a ratio of the fourth FT-IR peak area to the first FT-IR peak area of at least 0.25, which falls within a preferred range. On the other hand, the protein immobilization carrier hydrates of Comparative Examples 1 and 2 each had a ratio of the fourth FT-IR peak area to the first FT-IR peak area of less than 0.25, which is outside a preferred range.

Production of Protein-Carrying Complex

The protein immobilization carrier of Example 1 was dried at a temperature of 120°C for 12 hours. Subsequently, 10 mg of the dried protein immobilization carrier was mixed with 10 mL of a 1 mM phosphoric acid buffer solution. Next, the mixture was subjected to three times of mixing operation including application of ultrasonic vibration (frequency 40 kHz, power 360 W) for 5 minutes followed by shaking for 10 seconds. The resulting mixture was then agitated at a liquid temperature of 37°C for 3 hours. The mixture was then centrifuged at a liquid temperature of 4°C and a centrifugal force of 13000 $\times$ g for 30 minutes using a centrifuge (3700 (model number) manufactured by Kubota) so that the dispersoid was separated. After being washed with ultra-pure water, the resulting dispersoid was subjected again to centrifugation at a liquid temperature of 4°C and a centrifugal force of 13000 $\times$ g for 30 minutes so that the dispersoid was separated. The dispersoid was mixed with 10 mL of a 1 mM phosphoric acid buffer solution containing an IgG antibody (307-51131 (product number) manufactured by Wako Pure Chemical Industries) at a concentration of 1 mg/mL. The mixture was agitated at a liquid temperature of 37°C for 3 hours to give a protein-immobilized carrier dispersion. The protein-immobilized carrier dispersion was placed in a centrifuge tube and centrifuged at a liquid temperature of 4°C and a centrifugal force of 13000 × g for 30 minutes using a centrifuge so that the dispersoid was separated. After being washed with 10 mL of ultra-pure water, the resulting dispersoid was subjected again to centrifugation at a liquid temperature of 4°C and a centrifugal force of 13000 × g for 30 minutes so that the dispersoid was separated. The centrifuge tube containing the dispersoid was capped and then subjected to freeze drying under the conditions of -50°C and 3.0 Pa for 12 hours using a freeze dryer (FDU-1200 (model number) manufactured by Tokyo Rikakikai) so that a protein-carrying complex of Example 1 was obtained. The protein immobilization carriers of Example 2 and Comparative Example 1 were also subjected to the same procedure to produce protein-carrying complexes of Example 2 and Comparative Example 1.

[4-1] UV-Vis Analysis of Protein-Carrying Complex

For each of the protein-carrying complexes of Examples 1 and 2 and Comparative Example 1, a mixture solution of the supernatant liquid and the wash solution (after the final washing of the dispersoid) obtained during the production of the protein-carrying complex was prepared and measured for the intensity of maximum absorption at a wavelength (279 nm) attributed to aromatic amino acids using ultraviolet-visible spectroscopy (UV-Vis). The measurements were used to calculate the total concentration of the tyrosine, tryptophan, and phenylalanine residues (aromatic amino acid residues), from which the amount of the adsorbed protein was determined. The results are shown in Table 9.

[Table 9]

|  | Total concentration [$\mu$mol/g] of aromatic amino acids |
| --- | --- |
| Example 1 | 5.2 |
| Example 2 | 5.0 |
| Comparative Example 1 | 4.5 |

As a result, the protein-carrying complexes of Examples 1 and 2 each had a total concentration of tyrosine, tryptophan, and phenylalanine (aromatic amino acids) of at least 5.0 $\mu$mol/g, while the protein-carrying complex of Comparative Example 1 had a total aromatic amino acid concentration of 4.5 $\mu$mol/g. In particular, the protein immobilization carrier of Example 1 with a higher chlorine atom molar concentration provided a higher total aromatic amino acid concentration than the protein immobilization carrier of Example 2 with a lower chlorine atom molar concentration, which indicates that, regarding the protein-carrying complex of the present invention, the amount of protein adsorbable on the protein immobilization carrier of the present invention will increase with increasing chlorine atom molar concentration.

[4-2] Fourier Transform Infrared (FT-IR) Spectrophotometric Analysis of Protein-Carrying Complex

The protein-carrying complexes of Examples 1 and 2 and Comparative Examples 1 and 2 were analyzed by diffusion reflection method using a Fourier transform infrared spectrophotometer (FT-IR) (FT/IR-4600 (product name) manufactured by JASCO) and KBr (background) under the conditions below. More specifically, 49 mg of KBr and 1 mg of the protein-carrying complex were mixed to form a sample, which was subjected to the analysis under the conditions of the wavenumber range of 1750 cm$^{-1}$ to 1600 cm$^{-1}$ (corresponding to amide I absorption band), a number of scans of 128, and a spectroscopic resolution of 4 cm$^{-1}$. The amide I absorption band, which is derived from stretching vibration of C=O in proteins, is considered to be most sensitive to the secondary structure effect than vibration of other protein-derived amide groups. The measurement result was subjected to subtraction of the KBr background spectrum and then to KM (Kubelka-Munk) transformation, resulting in an FT-IR spectrum showing the intensity ratio proportional to the concentration.

In order to eliminate the effect of deformation vibration (at 1630 cm$^{-1}$) of O-H in water, the resulting FT-IR spectrum (after the adsorption of IgG) was overlaid on the FT-IR spectrum (before the adsorption of IgG) obtained for [1-3] Fourier Transform Infrared (FT-IR) Spectrophotometric Analysis of Protein Immobilization Carrier, and the difference between them was calculated to create an FT-IR spectrum (differential spectrum) for the molecular movement caused by the IgG adsorption. The resulting FT-IR spectra for Examples 1 and 2 and Comparative Examples 1 and 2 are shown in FIG. 8A, FIG. 8B, FIG. 8C, and FIG. 8D, respectively.

The amide I band of the IgG-adsorbed sample in the FT-IR spectrum in each of FIG. 8A, FIG. 8B, FIG. 8C, and FIG. 8D was subjected to secondary differentiation for peak assignment. The following peaks were separated from the spectrum and the areas of the following peaks were calculated: three FT-IR peaks at wavenumbers of 1619 $\pm$ 1.4 cm$^{-1}$,

1629 $\pm$ 0.8 cm$^{-1}$, and 1680 $\pm$ 0.3 cm$^{-1}$ derived from $\beta$-sheet (designated as $\beta$-sheet (1), $\beta$-sheet (2), and $\beta$-sheet (3), respectively); an FT-IR peak at a wavenumber of 1658 $\pm$ 0.3 cm$^{-1}$ derived from $\beta$-turn (1); an FT-IR peak at a wavenumber of 1689 $\pm$ 0.8 cm$^{-1}$ derived from $\beta$-turn (2); an FT-IR peak at a wavenumber of 1649 $\pm$ 1.1 cm$^{-1}$ derived from $\alpha$-helix; an FT-IR peak at a wavenumber of 1641 $\pm$ 0.2 cm$^{-1}$ derived from Random; and an FT-IR peak at a wavenumber of 1666 cm$^{-1}$ derived from Turn. The ratio of the total area of the four FT-IR peaks derived from $\alpha$-helix and $\beta$-sheet to the total area of the two FT-IR peaks derived from Random and Turn was calculated. The results are shown in Table 10. Peaks separated from the FT-IR spectra in FIG. 8A, FIG. 8B, FIG. 8C, and FIG. 8D are shown in FIG. 9A, FIG. 9B, FIG. 9C, and FIG. 9D, respectively.

[Table 10]

| | Area (a) of three FT-IR peaks derived from $\beta$-sheet | Area (b) of FT-IR peak derived from $\beta$-turn (1) | Area (c) of FT-IR peak derived from $\beta$-turn (2) | Area (d) of FT-IR peak derived from $\alpha$-helix | Area (e) of FT-IR peak derived from Random | Area (f) of FT-IR peak derived from Turn | Ratio ((d+a)/(e+f)) of the total area of four FT-IR peaks derived from $\alpha$-helix and $\beta$-sheet to the total area of two FT-IR peaks derived from Random and Turn |
|---|---|---|---|---|---|---|---|
| Example 1 | 40.432 | 5.5239 | 15.439 | 29.088 | 2.3808 | 7.1331 | 7.3 |
| Example 2 | 36.349 | 4.8340 | 19.809 | 26.924 | 3.9806 | 8.1027 | 5.0 |
| Comparative Example 1 | 27.024 | 5.0216 | 24.920 | 20.213 | 8.1792 | 14.642 | **2.0** |
| Comparative Example 2 | 31.391 | 4.7840 | 21.521 | 10.974 | 17.348 | 13.981 | **1.5** |
| (Note) Bold and underline indicate that the value is outside a preferred range of the present invention. The peak areas (a), (b), (c), (d), (e), and (f) are relative values calculated based on the total of the peak areas (a) to (f) normalized to 100%. | | | | | | | |

As a result, the protein-carrying complexes of Examples 1 and 2 each had a ratio of the total area of four FT-IR peaks derived from $\alpha$-helix and $\beta$-sheet to the total area of two FT-IR peaks derived from Random and Turn of at least 5.0, while the protein-carrying complexes of Comparative Examples 1 and 2 each had a low ratio of the total area of four FT-IR peaks derived from $\alpha$-helix and $\beta$-sheet to the total area of two FT-IR peaks derived from Random and Turn of 2.0. This indicates that the protein-carrying complex of Example 1 or 2 is less likely to cause denaturation of the protein adsorbed thereon than the protein-carrying complex of Comparative Example 1 or 2.

Summary

The protein immobilization carrier of Example 1 or 2 has a $(Q_2 + Q_3)/(Q_2 + Q_3 + Q_4)$ ratio of at most 0.30 as shown in the section [1-1] Solid-State $^{29}$Si-NMR Spectrum of Protein Immobilization Carriers; can adsorb a larger amount of a protein as shown in the section [4-1] UV-Vis Analysis of Protein-Carrying Complex when it has a chlorine atom molar concentration of at least 0.01 mol% as shown in the section [1-2] Chemical Composition Measured Using X-ray Fluorescence (XRF) Analysis of Protein Immobilization Carrier; and is less likely to cause denaturation of the protein adsorbed thereon as shown in the section [4-2] Fourier Transform Infrared (FT-IR) Spectrophotometric Analysis of Protein-Carrying Complex.

In addition, the protein immobilization carrier of Example 1 or 2 can be prepared using a simple method including reacting chlorine gas or chlorine compound gas such as silicon tetrachloride gas with a surface layer portion of a silica compound raw material including silicon (Si) and oxygen (O) to dope the silica compound with chlorine (Cl).

As described above, the present invention provides a protein immobilization carrier that can be produced using a simple method, is less likely to cause denaturation of proteins adsorbed thereon, and has a high ability to bind to proteins; a liquid dispersion of such a protein immobilization carrier; a hydrate of such a protein immobilization carrier; a protein-carrying complex including such a protein immobilization carrier; and an antibody test kit including such a protein immobilization carrier.

EXPLANATION OF REFERENCE NUMERALS

1: Protein immobilization carrier
2: Protein immobilization carrier hydrate
20: Water molecule
21: Hydrated layer
22: Hydrogen bonding
23: Buffer solution component
3: Protein-carrying complex
30: Protein
30': Denatured protein
30a: Hydrophilic terminal of protein
30b: Hydrophobic terminal of protein
4a, 4b: Target substance

**Claims**

1. A protein immobilization carrier comprising an amorphous silica-based compound including silicon (Si), oxygen (O), and chlorine (Cl),

   the protein immobilization carrier having a solid-state $^{29}$Si-NMR spectrum with a peak area $Q_4$ derived from $Si(OSi)_4$, a peak area $Q_3$ derived from $HO-Si(OSi)_3$, and a peak area $Q_2$ derived from $(HO)_2-Si(OSi)_2$ and with a $(Q_2 + Q_3) (Q_2 + Q_3 + Q_4)$ ratio of 0.30 or less,
   the protein immobilization carrier having a chlorine atom molar concentration of 0.01 mol% or more as measured using X-ray fluorescence (XRF) analysis.

2. The protein immobilization carrier according to claim 1, wherein the protein immobilization carrier has a Fourier transform infrared (FT-IR) spectrophotometric spectrum with a ratio of a peak area of a peak at a wavenumber of 2800 $cm^{-1}$ to the integral of absorbance in a wavenumber range of 3800 $cm^{-1}$ to 2500 $cm^{-1}$ of 0.005 or more.

3. The protein immobilization carrier according to claim 1, wherein the protein immobilization carrier is in the form of particles with a minimum particle size of 50 nm or more.

4. A protein immobilization carrier dispersion comprising: a dispersion medium comprising water; and the protein immobilization carrier according to any one of claims 1 to 3 dispersed in the dispersion medium.

5. The protein immobilization carrier dispersion according to claim 4, wherein the dispersion medium comprises a buffer solution.

6. The protein immobilization carrier dispersion according to claim 4, wherein the protein immobilization carrier has a surface zeta potential more positive than -20 mV.

7. A protein immobilization carrier hydrate comprising: the protein immobilization carrier according to any one of claims 1 to 3; and a hydrated layer on a surface of the protein immobilization carrier.

8. The protein immobilization carrier hydrate according to claim 7, wherein the protein immobilization carrier hydrate has a hydrated water weight concentration of 0.50% by weight or less based on the total weight of the carrier hydrate as determined using thermogravimetric-differential thermal analysis (TG-DTA).

9. The protein immobilization carrier hydrate according to claim 7,

   wherein the protein immobilization carrier hydrate has a Fourier transform infrared (FT-IR) spectrophotometric spectrum with
   a first FT-IR peak at a wavenumber of 3190 $\pm$ 20 $cm^{-1}$ derived from asymmetric stretching vibration of O-H in water molecules,
   a second FT-IR peak at a wavenumber of 3260 $\pm$ 20 $cm^{-1}$ derived from deformation vibration of O-H in water molecules,
   a third FT-IR peak at a wavenumber of 3430 $\pm$ 20 $cm^{-1}$ derived from symmetric stretching vibration of O-H in water molecules,

a fourth FT-IR peak at a wavenumber of 3480 $\pm$ 20 cm$^{-1}$ derived from stretching deformation vibration of water molecules on Si-OH, and

a fifth FT-IR peak at a wavenumber of 3610 $\pm$ 20 cm$^{-1}$ derived from stretching vibration of O-H in hydrogen-bonded water molecules and from asymmetric stretching vibration of water molecules on Si-OH,

wherein the first, second, third, fourth, and fifth FT-IR peaks are distinguishable from one another, and

wherein the Fourier transform infrared (FT-IR) spectrophotometric spectrum has a ratio of the area of the fourth FT-IR peak to the area of the first FT-IR peak of 0.25 or more.

10. A protein-carrying complex comprising: the protein immobilization carrier hydrate according to claim 7; and a protein adsorbed on the hydrated layer of the protein immobilization carrier hydrate.

11. An antibody test kit comprising a detection portion comprising at least the protein-carrying complex according to claim 10, wherein the protein is an antibody protein.

# FIG. 1A

# FIG. 1B

# FIG. 1C

EP 4 671 764 A1

# FIG. 2

FIG. 3A

FIG. 3B

EP 4 671 764 A1

FIG. 4A

EXAMPLE 1

FIG. 4B

EXAMPLE 2

FIG. 4C

COMPARATIVE
EXAMPLE 1

FIG. 5A

FIG. 5B

FIG. 5C

EP 4 671 764 A1

# FIG. 6A

# FIG. 6B

# FIG. 6C

# FIG. 6D

27

# FIG. 7A

# FIG. 7B

# FIG. 7C

# FIG. 7D

| | |
|---|---|
| —— DIFFERENTIAL SPECTRUM | -·-·- FIRST FT-IR PEAK |
| -··-··- SECOND FT-IR PEAK | ········ THIRD FT-IR PEAK |
| ----- FOURTH FT-IR PEAK | -- -- FIFTH FT-IR PEAK |
| ━━━ RECOMBINED VALUE | |

## FIG. 8A

## FIG. 8B

## FIG. 8C

## FIG. 8D

FIG. 9A

FIG. 9B

FIG. 9C

FIG. 9D

Legend:
- DIFFERENTIAL SPECTRUM
- $\beta$-sheet(3)
- $\beta$-turn(1)
- Random
- $\beta$-sheet(1)
- $\beta$-turn(2)
- Turn
- $\alpha$-helix
- $\beta$-sheet(2)
- RECOMBINED VALUE

## INTERNATIONAL SEARCH REPORT

| International application No. |
|---|
| **PCT/JP2023/042614** |

| A. | CLASSIFICATION OF SUBJECT MATTER |
|---|---|

*G01N 33/552*(2006.01)i; *B01J 20/24*(2006.01)i; *C01B 33/12*(2006.01)i; *C01B 37/00*(2006.01)i; *C07K 16/00*(2006.01)i; *C07K 17/14*(2006.01)i; *C12M 1/34*(2006.01)i

FI: G01N33/552; C01B37/00; B01J20/24 C; C12M1/34 F; C07K17/14; C07K16/00; C01B33/12 A

According to International Patent Classification (IPC) or to both national classification and IPC

| B. | FIELDS SEARCHED |
|---|---|

Minimum documentation searched (classification system followed by classification symbols)

G01N33/552; B01J20/24; C01B33/12; C01B37/00; C07K16/00; C07K17/14; C12M1/34

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2021-147255 A (OHARA INC.) 27 September 2021 (2021-09-27) | 1-5 |
| | claims, paragraphs [0041]-[0153], fig. 1-17 | |
| Y | | 6-7, 10-11 |
| Y | JP 07-270423 A (SEKISUI CHEMICAL CO., LTD.) 20 October 1995 (1995-10-20) claims 1-2, paragraphs [0021]-[0069] | 6 |
| Y | WO 2018/123952 A1 (NIPPON STEEL & SUMIKIN CHEMICAL CO., LTD.) 05 July 2018 (2018-07-05) paragraphs [0115]-[0166] | 6 |
| Y | WO 2014/203614 A1 (KONICA MINOLTA, INC.) 24 December 2014 (2014-12-24) paragraphs [0013]-[0185] | 6 |
| Y | JP 2022-529578 A (NANOLOGICA AB) 23 June 2022 (2022-06-23) paragraphs [0076]-[0096] | 7, 10-11 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 February 2024** | **13 February 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

# EP 4 671 764 A1

## INTERNATIONAL SEARCH REPORT

| International application No. |
|---|
| **PCT/JP2023/042614** |

### C. DOCUMENTS CONSIDERED TO BE RELEVANT

| Category* | Citation of document, with indication, where appropriate, of the relevant passages | Relevant to claim No. |
|---|---|---|
| Y | JP 2022-502534 A (SUMITOMO CHEMICAL COMPANY, LIMITED) 11 January 2022 (2022-01-11)<br>    paragraphs [0156]-[0160] | 7, 10-11 |
| Y | JP 2021-060403 A (TOSOH CORP.) 15 April 2021 (2021-04-15)<br>    paragraphs [0062]-[0066] | 7, 10-11 |
| A | JP 2017-179003 A (OHARA QUARTZ CO., LTD.) 05 October 2017 (2017-10-05)<br>    entire text, all drawings | 1-11 |
| A | TAGAYA, M. et al. Synthesis of Luminescent Nanoporous Silica Spheres Functionalized with Folic Acid for Targeting to C. Inorganic Chemistry. 12 June 2014, vol. 53, pp. 6817-6827, https://doi.org/10.1021/ic 500609g<br>    entire text, all drawings | 1-11 |

Form PCT/ISA/210 (second sheet) (July 2022)

32

## INTERNATIONAL SEARCH REPORT
### Information on patent family members

International application No.

**PCT/JP2023/042614**

| Patent document cited in search report | | | Publication date (day/month/year) | Patent family member(s) | | | Publication date (day/month/year) |
|---|---|---|---|---|---|---|---|
| JP | 2021-147255 | A | 27 September 2021 | US claims, paragraphs [0075]-[0203], fig. 1-17 | 2023/0142721 | A1 | |
| | | | | WO | 2021/187492 | A1 | |
| | | | | EP | 4122886 | A1 | |
| | | | | CN | 115362128 | A | |
| JP | 07-270423 | A | 20 October 1995 | (Family: none) | | | |
| WO | 2018/123952 | A1 | 05 July 2018 | US paragraphs [0158]-[0244] | 2019/0367694 | A1 | |
| | | | | EP | 3564675 | A1 | |
| | | | | CN | 110140053 | A | |
| | | | | KR | 10-2019-0101992 | A | |
| WO | 2014/203614 | A1 | 24 December 2014 | US paragraphs [0014]-[0193] | 2016/0178621 | A1 | |
| | | | | EP | 3012632 | A1 | |
| | | | | CN | 105324667 | A | |
| JP | 2022-529578 | A | 23 June 2022 | US paragraphs [0109]-[0114] | 2022/0202716 | A1 | |
| | | | | WO | 2020/212418 | A1 | |
| | | | | EP | 3894857 | B1 | |
| | | | | CN | 113454456 | A | |
| | | | | KR | 10-2021-0153080 | A | |
| JP | 2022-502534 | A | 11 January 2022 | US paragraphs [0177]-[0182] | 2021/0355379 | A1 | |
| | | | | WO | 2020/058440 | A1 | |
| | | | | EP | 3853322 | B1 | |
| | | | | CN | 112739792 | A | |
| JP | 2021-060403 | A | 15 April 2021 | (Family: none) | | | |
| JP | 2017-179003 | A | 05 October 2017 | US entire text, all drawings | 2019/0127635 | A1 | |
| | | | | WO | 2017/170531 | A1 | |
| | | | | EP | 3438227 | A1 | |
| | | | | CN | 109072070 | A | |

Form PCT/ISA/210 (patent family annex) (July 2022)

**REFERENCES CITED IN THE DESCRIPTION**

**Patent documents cited in the description**

- JP 2017047365 A
- JP 2018533543 A